# EUROPEAN PATENT APPLICATION

(11) **EP 3 714 898 A1**
(43) Date of publication of application: **30.09.2020**
(21) Application number: 20172302.0
(22) Date of filing: 17.07.2015
(51) Int. Cl.: A61K 39/00, A61K 35/12, A61K 35/14, A61K 35/28, A61K 39/395, A61K 39/385, C12N 5/00, C07K 1/00, C07K 2/00, C07K 14/00

(54) **DENDRITIC CELL VACCINES IN COMBINATION WITH THERAPY FOR BLOCKING HER2 AND HER3**

(30) Priority: 17.07.2014 US 201462025673 P; 17.07.2014 US 201462025685 P; 28.07.2014 US 201462028774 P; 22.05.2015 US 201562165445 P
(62) Divisional of application: 15822053.3
(71) Applicant: Czerniecki, Brian, J., Haddonfield, NJ 08033 (US)
(72) Inventor: CZERNIECKI, Brian, J., Haddonfield, NJ 08033 (US); KOSKI, Gary K., Akron, OH 44333 (US)
(74) Representative: Biggi, Cristina

(57) **Abstract**

The present invention relates to an FDA- approved injectable multi-dose antigen pulsed dendritic cell (DC) vaccine. In one embodiment, the activated antigen-loaded DC vaccine comprises an initial immunizing dose and multiple "booster" doses. The invention also provides a method of blocking both HER-2 and HER-3 as a treatment in causing permanent tumor senescence in HER-2 expressing breast cancers.

## Description

### BACKGROUND OF THE INVENTION

Dendritic cells (DCs) are white blood cells that acquire protein antigens from microbes or *even* cancerous cells and show, or "present" these antigens to T cells. The T cells, thus activated by the DCs, then initiate systemic immune responses to challenge the threat. Traditional vaccines against microbes contain additives known as "adjuvants" that by a number of possible means enhance DC activity within the vaccinated individual and amplify vaccine-induced immune responses. The requirements of vaccines against cancer, however, present a number of unique problems. For example, traditional adjuvants do not provide the proper signals to DCs that allow them to initiate optimal immunity against cancer. Also, the tumors themselves produce an environment that can affect the proper activation of DCs.

A popular solution to this problem is to extract DCs from cancer patients, load them with tumor antigens *in vitro,* and then supply unique activation signals to the cells before re-administering them to the body. This ensures proper DC activation removed from the influence of the tumor environment. When returned to the body, the DCs can then interact with T cells and initiate powerful anti-tumor immunity. Whereas the use of extra-corporealized DCs has solved many efficacy issues, it has historically come at the price of practical limitations. For example, since the DC vaccines are comprised of living cells, a special cell processing and vaccine production facility has been required at the physical location of the medical center administering the therapy. This is an expensive and inefficient way to deliver the therapy because every institution administering such treatment would have to build and maintain their own special-use facility.

Management of breast cancer currently relies on a combination of early diagnosis and aggressive treatment, which can include one or more treatments such as surgery, radiation therapy, chemotherapy, and hormone therapy. Herceptin (trastuzumab) was developed as a targeted therapy for HER2/ErbB2 positive breast cancer cells, often used in conjunction with other therapies, including the mitotic inhibitor paclitaxel (sold under the trade name Taxol).

The efficacy of Herceptin as a monotherapy is estimated to be less than 30%; combinatorial treatment with microtubule stabilizing drugs such as paclitaxel increases efficacy to approximately 60% (Burris et al., 2000, Semin Oncol 27: 19-23). Treatment with Herceptin results in accumulation of the Cdk inhibitor p27 and subsequent G1/S cell cycle arrest, and paclitaxel stalls the entry of mitosis which can lead to cell death. In spite of great promise, however, high doses of Herceptin or paclitaxel result in undesirable side effects. Further, the cancer often develops resistance to Herceptin and/or paclitaxel.

Therefore, there remains an unmet need for compositions and efficient methods for producing maximal therapeutic DC vaccines and for new methods of treating cancer using Herceptin. Accordingly, there is a need in the art to have additional immunotherapeutic approaches for treating or preventing breast cancer and other malignancies. The present invention fulfills this need.

### BRIEF DESCRIPTION OF THE DRAWINGS

The following detailed description of preferred embodiments of the invention will be better understood when read in conjunction with the appended drawings. For the purpose of illustrating the invention, there are shown in the drawings embodiments which are presently preferred. It should be understood, however, that the invention is not limited to the precise arrangements and instrumentalities of the embodiments shown in the drawings.

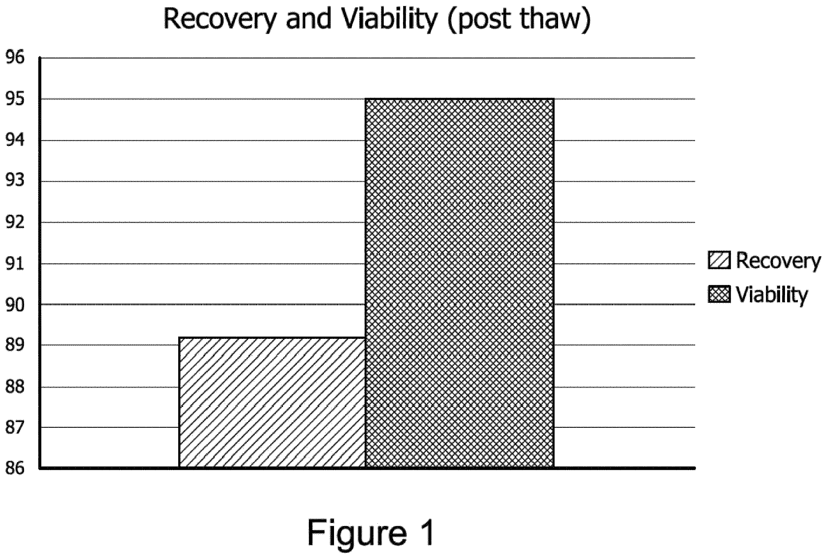

Figure 1 is a chart showing the viability and yield of cryopreserved DC1. Recovery of cells was on average 89% and viability was 95% when cells were directly thawed and counted.

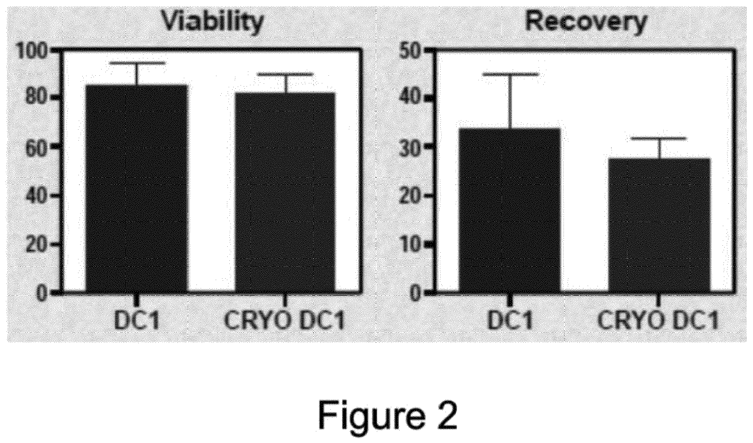

Figure 2 is a chart showing that here was no significant difference in the viability (p=.4807), and recovery (p=.1220) of the cells.

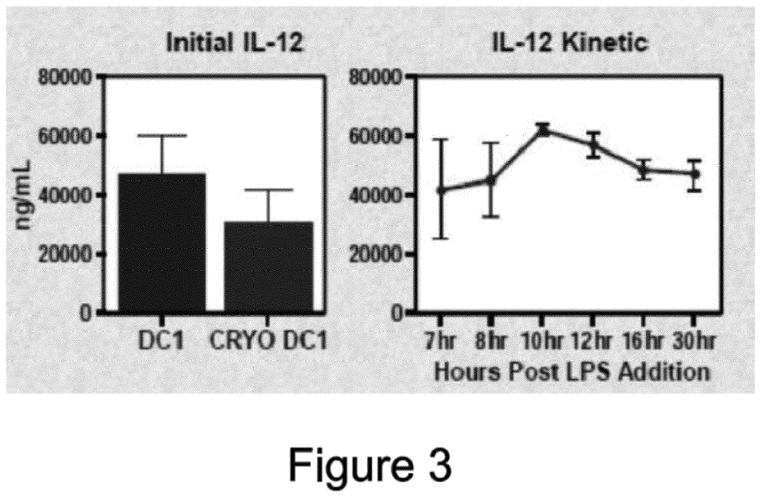

Figure 3 is a chart showing that both populations had similar initial (7 hours post LPS addition) IL-12 p70 secretion (p=.0768). The populations continued to exhibit comparable secretion levels of IL-12 p70 over a 30 hour observation period with no significant differences between the populations.

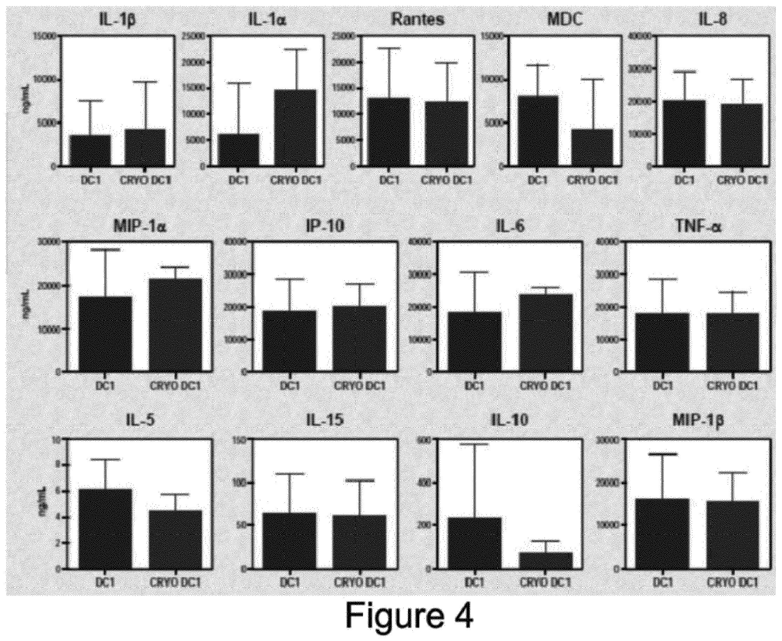

Figure 4 is a chart showing that there was no significant difference between populations and production of IL-1beta (p=0.7690), IL-1 alpha (p=0.0841), Rantes (p=0.902), MDC (p=0.1514), IL-10 (p=.1937), MIP-1alpha (p=.2673), IP-10 (p=0.7366), IL-6 (p=0.24), IL-5 (p=0.0735), TNF-beta (p=0.9422), IL-15(p=0.8878), MIP-1beta (p=0.9217), TNF-alpha (p=0.8972), IL-8(p=0.7844).

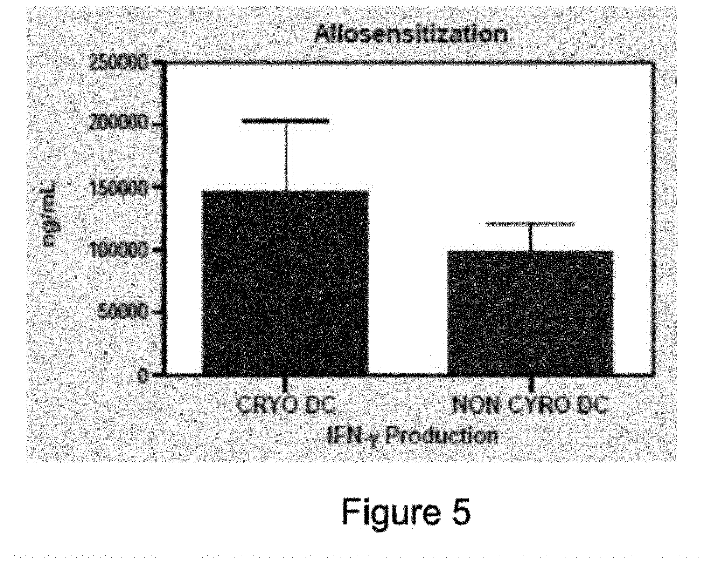

Figure 5 is a chart showing production of IFN-γ from cryopreserved and non-cryopreserved DCs.

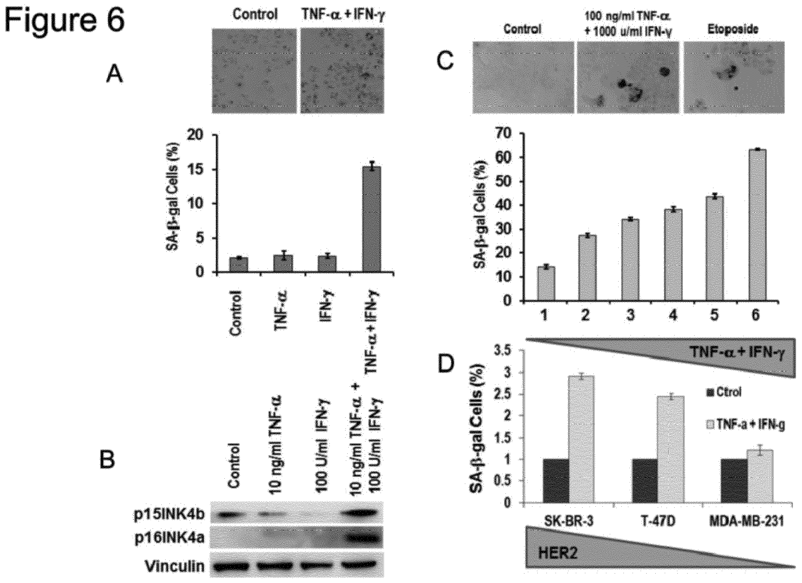

Figure 6. Th1 cytokines TNF-α and IFN-γ synergize to Induce senescence in breast cancer cells and the doses required are in an inverse correlation with the HER2 expression. Figure 6A. SK-BR-3 breast cancer line was incubated with 10 ng/ml TNF-α and 100 U/ml IFN-γ for 5 days, cultured for 2 more passages in absence of cytokines and then stained for SA-β-galactosidase (SA-β-gal) expression (senescence marker) and compared to untreated control cells. Only paired cytokines induced senescence. *Top panel,* representative data from 1 of 3 independent experiments. *Bottom panel,* densitometric analysis. Data are presented as % of SA-β-gal-positive cells and presented as mean + S.D. (n=3). Figure 6B. Cell lysates of the cells described in A were analyzed by western blotting for p151NKb and p16INK4a expression. Vinculin was used as loading control. Figure 6C. T-47D breast cancer cells were untreated (1) or incubated with the following concentrations of TNF-α and INF-γ: 10 ng/ml and 100 U/ml (2), 50 ng/ml and 500 U/ml (3), 75 ng/ml and 750 U/ml (5), 100 ng/ml and 1000 U/ml (5) for 5 days and cultured for 2 more passages in absence of cytokines. The cells were then stained for SA-β-gal and compared to control untreated cells or those treated with 8 uM etoposide as positive control (6). *Top panel,* representative data from 1 of 3 independent experiments. *Bottom panel,* densitometric analysis. Data are presented as % of SA-β-ga1-positive cells and presented as mean ± S.D. (n=3). Figure 6D. Combination treatment with Th1 cytokines IFN-γ and TNF-α resulted in greater senescence in SK-BR-3 (10 ng/ml TNF-α + 100 U/ml IFN-γ) and T-47D (100 ng/ml TNF-α + 1000 U/ml IFN-γ) cells, compared with untreated controls; MDA-MB-231 cells (200 ng/ml TNF-α + 2000 U/ml IFN-γ) remained largely unaffected by dual IFN-γ + TNF-α treatment. Data are presented as % of SA-β-gal-positive cells and presented as mean + S.D. (n=3).

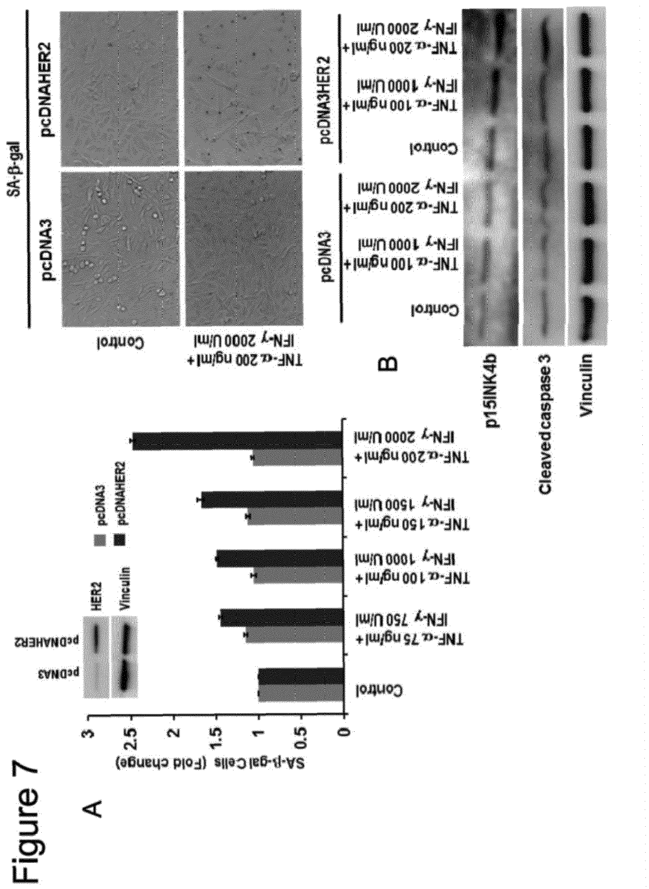

Figure 7. HER2 induces senescence and apoptosis in MDA-MB-231 breast cancer cells. Figure 7A. SA-β-gal staining was performed in MDA-MB-231 cells transfected with wt HER2 (pcDNAHER2) or with empty vector (pcDNA3) treated with the concentrations listed of TNF-α and IFN-γ for 5 days and cultured for 2 more passages in absence of cytokines *Left panel,* densitometric analysis. Data are presented as % of SA-β-gal-positive cells and presented as mean ± S.D. (n=3). *Right panel,* representative data from 1 of 3 independent experiments. Figure 7B. Cell lysates of the cells described in A were analyzed by western blotting for p151NKb expression and cleaved caspase-3 expression. Vinculin was used as loading control. Inset. Western blot of MDA-MB-231 cells stable transfected with pcDNAHER2 or pcDNA3 probed with HER2 specific antibody. Vinculin was used as loading control. Similar results were observed in 3 independent experiments.

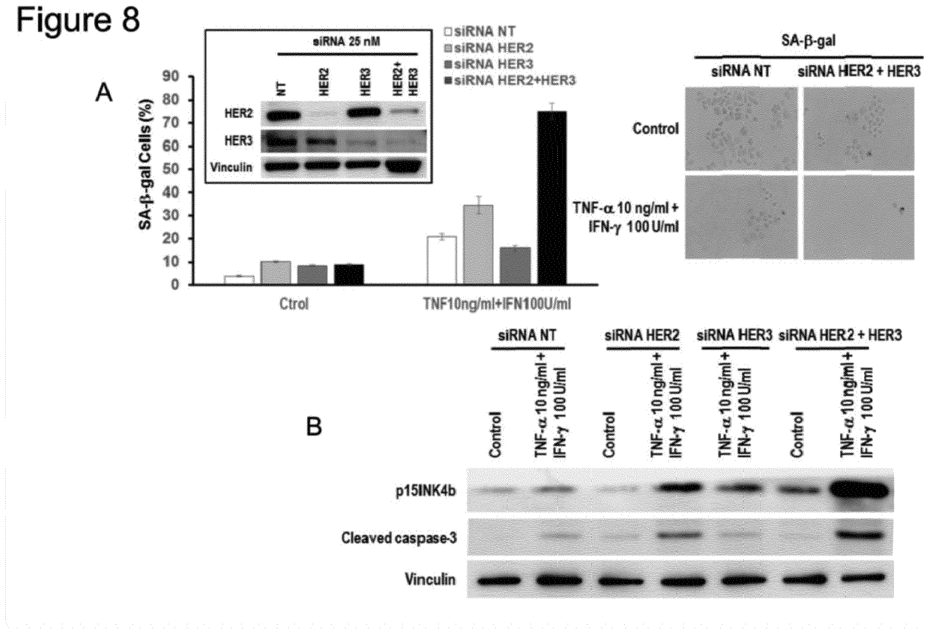

Figure 8. Combined HER2 and HER3 blockage expression enhances Th1 cytokines TNF-α and IFN-γ senescence induction and apoptosis in SK-BR-3 breast cancer cells. Figure 8A. SA-β-gal staining was performed in SK-BR-3 cells transfected with non-target (NT), HER2, HER3 or a combination of HER2 and HER3 siRNA, and then treated with the concentrations listed of TNF-α and IFN-γ for 5 days and cultured for 2 more passages in absence of cytokines. *Left panel,* densitometric analysis. Data are presented as % of SA-β-gal-positive cells and presented as mean + S.D. (n=3). *Right panel,* representative data from 1 of 3 independent experiments. Figure 8B. Cell lysates of the cells described in A were analyzed by western blotting for p151NKb or cleaved caspase-3 expression. Inset. Western blot of SK-BR-3 cells transfected with NT, HER2 or HER3 siRNA probed with HER2 and HER3 specific antibodies. Vinculin was used as loading control. Similar results were observed in 3 independent experiments.

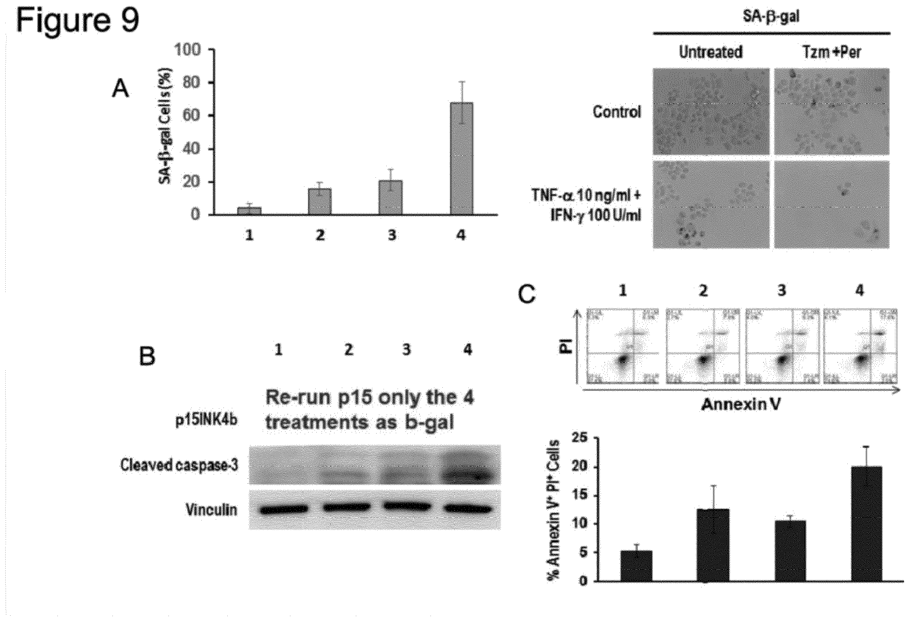

Figure 9. Combined HER2 inhibition and HER2-HER3 dimerization inhibition enhances Th1 cytokines TNF-α and IFN-γ senescence induction and apoptosis in SK-BR-3 breast cancer cells. Figure 9A. SA-β-gal staining was performed in SK-BR-3 cells untreated (1) or treated with 10 ng/ml TNF-α and 100 U/ml IFN-γ (2), or with 10 ug/ml of trastuzumab (Tzm), pertuzumab (Per)(3), or with the combination of both treatments (4) for 5 days and cultured for 2 more passages in absence of the antibodies and the cytokines. *Left panel,* densitometric analysis. Data are presented as % of SA-β-gal-positive cells and presented as mean + S.D. (n=3). *Right panel,* representative data from 1 of 3 independent experiments. Figure 9B. Cell lysates of the cells described in A were analyzed by western blotting for p151NKb or cleaved caspase-3 expression. Vinculin was used as loading control. Similar results were observed in 3 independent experiments. Figure 9C. Induction of apoptosis of SK-BR-3 cells untreated or treated as described above was performed by staining for annexin V and PI and analyzed by flow cytometry. *Top panel,* plots are representative data from 1 of 3 independent experiments. *Bottom panel,* densitometric analysis. Data are presented as average ± SEM of annexin V⁺ PI⁺ cells from 3 independent experiments.

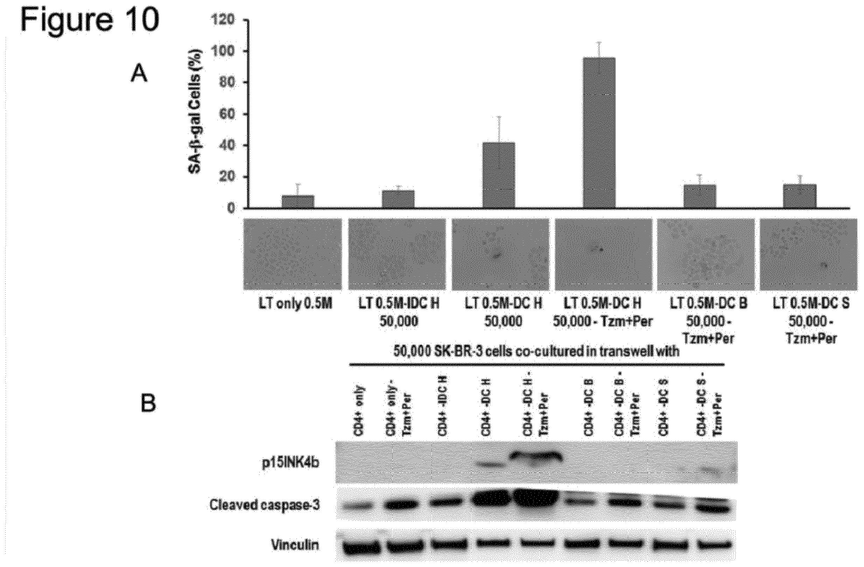

Figure 10. Combined treatment with trastuzumab and pertuzumab enhance CD4⁺ Th1-mediated senescence and apoptosis of HER2-ovexpressing human breast cancer cells. Figure 10A. Using a transwell system, 0.5x10⁵ SK-BR-3 cells were co-cultured with 5x10⁵ CD4⁺ T-cells alone (CD4⁺ only), CD4⁺ T-cells + 0.5x10⁵ each of HER2 Class II peptide (DC H)- or irrelevant Class II BRAF or survivin peptides (DC B or DC S)-pulsed type 1 polarized mature DCs, and CD4⁺ T-cells + HER2 (iDC H)-pulsed immature DCs, with or without 10 ug/ml of trastuzumab (Tzm) and pertuzumab (Per) for 5 days W. The cells were then cultured for 2 more passages in absence of the blocking antibodies and the immune system cells and then stained for SA-β-gal expression and compared to untreated control cells. *Top panel,* densitometric analysis. Data are presented as % of SA-β-gal-positive cells and presented as mean + S.D. (n=3). *Bottom panel,* representative data from 1 of 3 independent experiments. Figure 10B. Increased p15INK4b and cleaved caspase-3 expression suggests induced senescence and apoptosis of SK-BR-3 cells, respectively when co-cultured with the DC H/CD4⁺ T-cells in presence of trastuzumab and pertuzumab, but not from DC B, DC S and iDC H groups. Vinculin was used as loading control. Results are representative of 3 independent experiments.

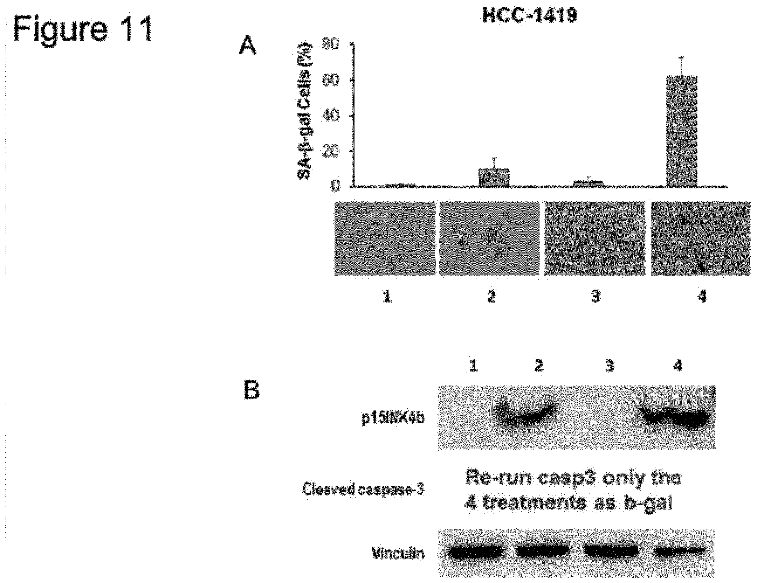

Figure 11. Th1 cytokines TNF-α and IFN-γ sensitize trastuzumab and pertuzumab resistant breast cancer cells to senescence and apoptosis induction. Figure 11A. SA-β-gal staining was performed in HCC-1419 and JIMT-1 cells, respectively untreated (1) or treated with 50 ng/ml TNF-α and 500 U/ml IFN-γ (2), or treated with 10 ug/ml of trastuzumab (Tzm), pertuzumab (Per) (3), or treated with the combination of the same concentrations of trastuzumab, pertuzumab and TNF-α, IFN-γ (4) for 5 days and cultured for 2 more passages in absence of the antibodies and the cytokines. *Top panel,* densitometric analysis. Data are presented as % of SA-β-gal-positive cells and presented as mean ± S.D. (n=3). *Bottom panel,* representative data from 1 of 3 independent experiments. Figure 11B. Cell lysates of the cells described in Figure 11A were analyzed by western blotting for p151NKb or cleaved caspase-3 expression. Vinculin was used as loading control. Similar results were observed in 3 independent experiments.

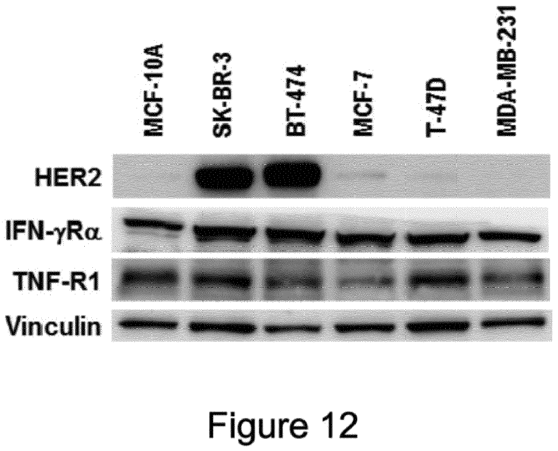

Figure 12. IFNGR and TNFR are expressed in similar levels in breast cell lines independently from their HER2 level. IFNGR, TNFR and HER2 expression in immortalized MCF-10A mammary epithelial cells and breast cancer cell lines (SK-BR-3, BT-474, MCF-7, T-47D and MDA-MB-231) as determined by Western blot. Vinculin was used as loading control. Similar results were observed in 3 independent experiments.

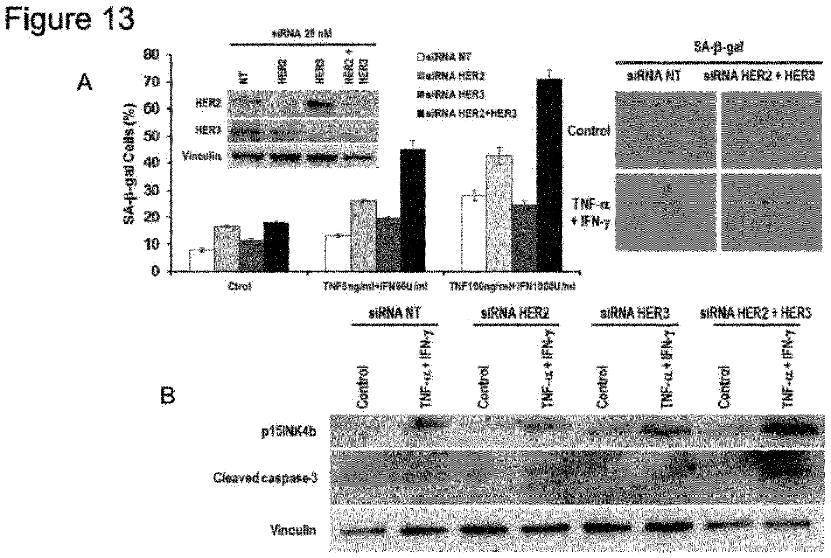

Figure 13. Combined HER2 and HER3 blockage expression enhances Th1 cytokines TNF-α and IFN-γ senescence induction in MCF-7 breast cancer cells. Figure 13A. SA-β-gal staining was performed in MCF-7 cells transfected with non-target (NT), HER2, HER3 or a combination of HER2 and HER3 siRNA, and then treated with the concentrations listed of TNF-α and IFN-γ for 5 days and cultured for 2 more passages in absence of cytokines. *Left panel,* densitometric analysis. Data are presented as % of SA-β-gal-positive cells and presented as mean + S.D. (n=3). *Right panel,* representative data from 1 of 3 independent experiments. Figure 13B. Cell lysates of the cells described in A were analyzed by western blotting for p151NKb or cleaved caspase-3 expression. Inset. Western blot of MCF-7 cells transfected with NT, HER2 or HER3 or a combination of HER2 and HER3 siRNA probed with HER2 and HER3 specific antibodies. Vinculin was used as loading control. Similar results were observed in 3 independent experiments.

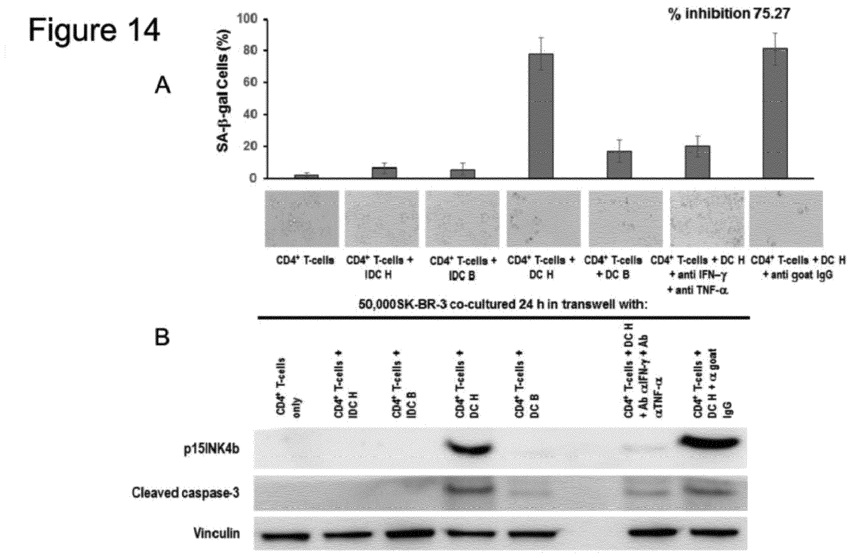

Figure 14. Effect of Th1-elaborated cytokines on SK-BR-3 senescence and apoptosis. Figure 14A. Using a transwell system, 0.5x10⁵ SK-BR-3 cells were co-cultured with 5x10⁵ CD4⁺ T-cells alone (CD4⁺ only), CD4⁺ T-cells + 0.5x10⁵ each of HER2 Class II peptide (DC H)- or irrelevant Class II BRAF peptide (DC B)-pulsed type 1 polarized mature DCs, and CD4⁺ T-cells + HER2 (iDC H)- or BRAF (iDC B)-pulsed immature DCs for 5 days. The cells were then cultured for 2 more passages in absence of immune system cells and then stained for SA-β-gal expression and compared to untreated control cells. Compared with IgG isotype control, senescence induced in SK-BR-3 treated with CD4⁺/DC H is partially rescued by neutralizing IFN-γ and TNF-α with specific antibodies (75.27 % rescue). *Top panel,* densitometric analysis. Data are presented as % of SA-β-gal-positive cells and presented as mean ± S.D. (n=3). *Bottom panel,* representative data from 1 of 3 independent experiments. Figure 14B. Increased p15INK4b and cleaved caspase 3 expressions suggest senescence and apoptosis induction of SK-BR-3 cells when co-cultured with DC H/CD4⁺ T-cells compared with DC B, iDC H, and iDC B groups. Compared with IgG isotype control, senescence and apoptosis induced in SK-BR-3 treated with CD4⁺/DC H were partially rescued by neutralizing IFN-γ and TNF-α specific antibodies. Vinculin was used as loading control. Results are representative of 3 experiments.

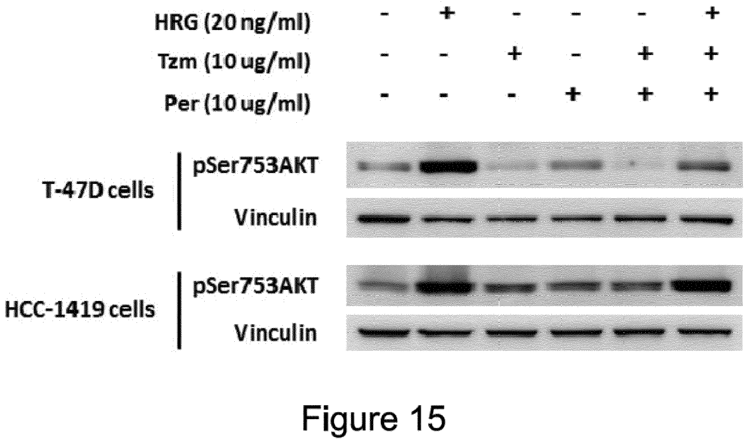

Figure 15. Effect of trastuzumab and pertuzumab on AKT activation by heregulin in breast cancer cell lines. Figure 16A. Serum-starved T-47D, HCC-1419 and JIMT-1 cells were treated with trastuzumab (Tzm) and pertuzumab (Per 10 ug/ml, 90 min) and then stimulated with (HRG, 20 ng/ml, 5 min). *Top panel,* representative data from 1 of 3 independent experiments. *Bottom panel,* densitometric analysis. Data are expressed as % of the HRG response in the absence of trastuzumab and pertuzumab and presented as mean ± S.D. (n=3).

### DETAILED DESCRIPTION

The present invention provides compositions and methods for producing an FDA-approved injectable multi-dose antigen pulsed dendritic cell vaccine for the personalized treatment and prevention of cancer or other disorders. In one embodiment, the invention provides compositions and methods for producing an FDA-approved injectable multi-dose antigen pulsed type I polarized dendritic cell vaccine (DC1).

In one embodiment, the invention provides a method to cryopreserve dendritic cells in multiple-dose aliquots that are in an antigen-loaded, pre-activated state that is "syringe-ready", i.e. suitable for immediate injection into the patient without the necessity of any further cell processing that would require (e.g., by FDA mandate) additional facilities and quality control/assurance steps.

In one embodiment, the invention provides a method to efficiently produce injectable multi-dose antigen pulsed dendritic cell vaccine, preferably injectable multi-dose antigen pulsed type I polarized dendritic cell vaccine that exhibit maximal efficacy.

In one embodiment, an FDA-approved injectable multi-dose antigen pulsed dendritic cell vaccine is produced by collecting DCs in a single patient leukapheresis. Preferably, the leukapheresis and production of the dendritic cell vaccine is performed at a first location whereby the first location can be a centralized vaccine production facility where the DCs are manipulated to create an activated antigen-loaded DC vaccine comprised of an initial immunizing dose and multiple "booster" doses thereof. An advantage of the present invention is that all FDA mandated quality control/quality assurance steps would be performed at the central facility, and after completion and release, all vaccine doses are cryopreserved and shipped to remote medical centers for serial administration to the patient. In one embodiment, the FDA-approved injectable multi-dose antigen pulsed dendritic cell vaccine of the invention does not requirement any mandated quality control/quality assurance steps at the administration site.

In another aspect, the present invention is based on the discovery that an effective therapy to treat cancer includes changing the immune response in the tumor so that the immune cells in the tumor site are more effective in attacking the tumor cells. In some instances, the effective therapy includes improving the migration and activity of immune cells in the tumor site. Accordingly, the invention provides compositions and methods of using a dendritic cell vaccine in combination with a composition that inhibits one or more of HER-2 and HER-3 (e.g., trastuzumab, pertuzumab, and the like) as a treatment regimen to treat cancer. In one embodiment, the treatment regimen comprises the use of dendritic cell vaccines, an inhibitor of HER-2, and a chemokine modulator.

In one embodiment, the invention provides compositions and methods for using a dendritic cell vaccine in combination with blockage of one or more of HER-2 and HER-3 as a treatment regimen to treat cancer. In another embodiment, the invention provides compositions and methods of using a dendritic cell vaccine in combination with blockage of HER-2 and HER-3 with the addition of TNF-α and IFN-γ. In another embodiment, the invention provides compositions and methods of blocking one or more of HER-2 and HER-3 with the addition of TNF-α and IFN-γ as a treatment regimen to treat cancer.

In one embodiment, the treatment regimen of the invention comprises a combination therapy of inducing an anti-oncodriver Th1 immune response (e.g., TNF-α and IFN-γ) and oncodriver blockade for one or more of HER-2 and HER-3

In one embodiment, the treatment regimen of the invention can be used to treat cancer and therefore can be considered as a type of anti-cancer therapy. In another embodiment, the treatment regimen of the invention can be used in combination with another anti-cancer therapy including but is not limited to surgery, chemotherapy, radiation therapy (e.g. X ray), gene therapy, immunotherapy, hormone therapy, viral therapy, DNA therapy, RNA therapy, protein therapy, cellular therapy, nanotherapy, and the like.

In one embodiment, the treatment regimen of the invention is used prior to receiving the other anti-cancer therapy. In another embodiment, the treatment regimen of the invention is used concurrently with receiving the other anti-cancer therapy. In another embodiment, the treatment regimen of the invention is used after receiving the other anti-cancer therapy.

### Definitions

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described.

Generally, the nomenclature used herein and the laboratory procedures in cell culture, molecular genetics, organic chemistry, and nucleic acid chemistry and hybridization are those well-known and commonly employed in the art.

Standard techniques are used for nucleic acid and peptide synthesis. The techniques and procedures are generally performed according to conventional methods in the art and various general references (e.g., Sambrook and Russell, 2012, Molecular Cloning, A Laboratory Approach, Cold Spring Harbor Press, Cold Spring Harbor, NY, and Ausubel et al., 2012, Current Protocols in Molecular Biology, John Wiley & Sons, NY), which are provided throughout this document.

The nomenclature used herein and the laboratory procedures used in analytical chemistry and organic syntheses described below are those well-known and commonly employed in the art. Standard techniques or modifications thereof are used for chemical syntheses and chemical analyses.

As used herein, each of the following terms has the meaning associated with it in this section.

The articles "a" and "an" are used herein to refer to one or to more than one (*i.e.,* to at least one) of the grammatical object of the article. By way of example, "an element" means one element or more than one element.

"About" as used herein when referring to a measurable value such as an amount, a temporal duration, and the like, is meant to encompass variations of ±20%, or ±10%, or ±5%, or ±1%, or ±0.1% from the specified value, as such variations are appropriate to perform the disclosed methods.

The term "abnormal" when used in the context of organisms, tissues, cells or components thereof, refers to those organisms, tissues, cells or components thereof that differ in at least one observable or detectable characteristic (e.g., age, treatment, time of day, etc.) from those organisms, tissues, cells or components thereof that display the "normal" (expected) respective characteristic. Characteristics which are normal or expected for one cell or tissue type, might be abnormal for a different cell or tissue type.

The term "antigen" or "ag" as used herein is defined as a molecule that provokes an immune response. This immune response may involve either antibody production, or the activation of specific immunologically-competent cells, or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequences or a partial nucleotide sequence encoding a protein that elicits an immune response therefore encodes an "antigen" as that term is used herein. Furthermore, one skilled in the art will understand that an antigen need not be encoded solely by a full length nucleotide sequence of a gene. It is readily apparent that the present invention includes, but is not limited to, the use of partial nucleotide sequences of more than one gene and that these nucleotide sequences are arranged in various combinations to elicit the desired immune response. Moreover, a skilled artisan will understand that an antigen need not be encoded by a "gene" at all. It is readily apparent that an antigen can be generated synthesized or can be derived from a biological sample. Such a biological sample can include, but is not limited to a tissue sample, a tumor sample, a cell or a biological fluid.

"An antigen presenting cell" (APC) is a cell that are capable of activating T cells, and includes, but is not limited to, monocytes/macrophages, B cells and dendritic cells (DCs).

"Antigen-loaded APC" or an "antigen-pulsed APC" includes an APC, which has been exposed to an antigen and activated by the antigen. For example, an APC may become Ag-loaded *in vitro,* e.g., during culture in the presence of an antigen. The APC may also be loaded *in vivo* by exposure to an antigen. An "antigen-loaded APC" is traditionally prepared in one of two ways: (1) small peptide fragments, known as antigenic peptides, are "pulsed" directly onto the outside of the APCs; or (2) the APC is incubated with whole proteins or protein particles which are then ingested by the APC. These proteins are digested into small peptide fragments by the APC and are eventually transported to and presented on the APC surface. In addition, the antigen-loaded APC can also be generated by introducing a polynucleotide encoding an antigen into the cell.

The term "autoimmune disease" as used herein is defined as a disorder that results from an autoimmune response. An autoimmune disease is the result of an inappropriate and excessive response to a self-antigen. Examples of autoimmune diseases include but are not limited to, Addision's disease, alopecia areata, ankylosing spondylitis, autoimmune hepatitis, autoimmune parotitis, Crohn's disease, diabetes (Type I), dystrophic epidermolysis bullosa, epididymitis, glomerulonephritis, Graves' disease, Guillain-Barr syndrome, Hashimoto's disease, hemolytic anemia, systemic lupus erythematosus, multiple sclerosis, myasthenia gravis, pemphigus vulgaris, psoriasis, rheumatic fever, rheumatoid arthritis, sarcoidosis, scleroderma, Sjogren's syndrome, spondyloarthropathies, thyroiditis, vasculitis, vitiligo, myxedema, pernicious anemia, ulcerative colitis, among others.

As used herein, the term "autologous" is meant to refer to any material derived from the same individual to which it is later to be re-introduced into the individual.

The term "B cell" as used herein is defined as a cell derived from the bone marrow and/or spleen. B cells can develop into plasma cells which produce antibodies.

The term "cancer" as used herein is defined as a hyperproliferation of cells whose unique trait--loss of normal control--results in unregulated growth, lack of differentiation, local tissue invasion, and/or metastasis. Examples include but are not limited to, breast cancer, prostate cancer, ovarian cancer, cervical cancer, skin cancer, pancreatic cancer, colorectal cancer, renal cancer and lung cancer.

The term "cryopreserved" or "cryopreservation" as used herein refers to cells that have been resuspended in a freezing medium and frozen at a temperature of around -70°C or lower.

The term "freezing medium" as used herein refers to any medium mixed with a cell sample in preparation for freezing, such that at least some of cells within the cell sample can be recovered and remain viable after thawing.

The term "dendritic cell" (DC) is an antigen presenting cell existing in vivo, in vitro, ex vivo, or in a host or subject, or which can be derived from a hematopoietic stem cell or a monocyte. Dendritic cells and their precursors can be isolated from a variety of lymphoid organs, e.g., spleen, lymph nodes, as well as from bone marrow and peripheral blood. The DC has a characteristic morphology with thin sheets (lamellipodia) extending in multiple directions away from the dendritic cell body. Typically, dendritic cells express high levels of MHC and costimulatory (e.g., B7-1 and B7-2) molecules. Dendritic cells can induce antigen specific differentiation of T cells in vitro, and are able to initiate primary T cell responses in vitro and in vivo.

As used herein, an "activated DC" is a DC that has been exposed to a Toll-like receptor agonist. The activated DC may or may not be loaded with an antigen.

The term "mature DC" as used herein, is defined as a dendritic cell that expresses molecules, including high levels of MHC class II, CD80 (B7.1) and CD86 (B7.2). In contrast, immature dendritic cells express low levels of MHC class II, CD80 (B7.1) and CD86 (B7.2) molecules, yet can still take up an antigen. "Mature DC" also refers to an antigen presenting cell existing in vivo, in vitro, ex vivo, or in a host or subject that is DC1-polarized (i.e., fully capable of promoting cell-mediated immunity).

A "disease" is a state of health of an animal wherein the animal cannot maintain homeostasis, and wherein if the disease is not ameliorated then the animal's health continues to deteriorate.

A "disorder" in an animal is a state of health in which the animal is able to maintain homeostasis, but in which the animal's state of health is less favorable than it would be in the absence of the disorder. Left untreated, a disorder does not necessarily cause a further decrease in the animal's state of health.

A disease or disorder is "alleviated" if the severity or frequency of at least one sign or symptom of the disease or disorder experienced by a patient is reduced.

"Effective amount" or "therapeutically effective amount" are used interchangeably herein, and refer to an amount of a compound, formulation, material, or composition, as described herein effective to achieve a particular biological result. Such results may include, but are not limited to, the inhibition of virus infection as determined by any means suitable in the art.

As used herein "endogenous" refers to any material from or produced inside an organism, cell, tissue or system.

As used herein, the term "exogenous" refers to any material introduced from or produced outside an organism, cell, tissue or system.

"Estrogen receptor (ER) positive" cancer is cancer which tests positive for expression of ER. Conversely, "ER negative" cancer tests negative for such expression. Analysis of ER status can be performed by any method known in the art.

A "HER receptor" is a receptor protein tyrosine kinase which belongs to the HER receptor family and includes EGFR (ErbB1, HER1), HER2 (ErbB2), HER3 (ErbB3) and HER4 (ErbB4) receptors. The HER receptor will generally comprise an extracellular domain, which may bind an HER ligand and/or dimerize with another HER receptor molecule; a lipophilic transmembrane domain; a conserved intracellular tyrosine kinase domain; and a carboxyl-terminal signaling domain harboring several tyrosine residues which can be phosphorylated. The HER receptor may be a "native sequence" HER receptor or an "amino acid sequence variant" thereof. Preferably the HER receptor is a native sequence human HER receptor.

The "HER pathway" refers to the signaling network mediated by the HER receptor family.

"HER activation" refers to activation, or phosphorylation, of any one or more HER receptors. Generally, HER activation results in signal transduction (e.g. that caused by an intracellular kinase domain of a HER receptor phosphorylating tyrosine residues in the HER receptor or a substrate polypeptide). HER activation may be mediated by HER ligand binding to a HER dimer comprising the HER receptor of interest. HER ligand binding to a HER dimer may activate a kinase domain of one or more of the HER receptors in the dimer and thereby results in phosphorylation of tyrosine residues in one or more of the HER receptors and/or phosphorylation of tyrosine residues in additional substrate polypeptides(s), such as Akt or MAPK intracellular kinases.

The term "hyperproliferative disease" is defined as a disease that results from a hyperproliferation of cells. Exemplary hyperproliferative diseases include, but are not limited to, cancer or autoimmune diseases. Other hyperproliferative diseases may include vascular occlusion, restenosis, atherosclerosis, or inflammatory bowel disease, for example.

The term "inhibit," as used herein, means to suppress or block an activity or function, for example, about ten percent relative to a control value. Preferably, the activity is suppressed or blocked by 50% compared to a control value, more preferably by 75%, and even more preferably by 95%. "Inhibit," as used herein, also means to reduce a molecule, a reaction, an interaction, a gene, an mRNA, and/or a protein's expression, stability, function or activity by a measurable amount or to prevent entirely. Inhibitors are compounds that, e.g., bind to, partially or totally block stimulation, decrease, prevent, delay activation, inactivate, desensitize, or down regulate a protein, a gene, and an mRNA stability, expression, function and activity, e.g., antagonists.

As used herein, an "instructional material" includes a publication, a recording, a diagram, or any other medium of expression which can be used to communicate the usefulness of the compositions and methods of the invention. The instructional material of the kit of the invention may, for example, be affixed to a container which contains the nucleic acid, peptide, and/or composition of the invention or be shipped together with a container which contains the nucleic acid, peptide, and/or composition. Alternatively, the instructional material may be shipped separately from the container with the intention that the instructional material and the compound be used cooperatively by the recipient.

"Isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

By the term "modulating," as used herein, is meant mediating a detectable increase or decrease in the level of a response in a subject compared with the level of a response in the subject in the absence of a treatment or compound, and/or compared with the level of a response in an otherwise identical but untreated subject. The term encompasses perturbing and/or affecting a native signal or response thereby mediating a beneficial therapeutic response in a subject, preferably, a human.

As used herein, a "population" includes reference to an isolated culture comprising a homogenous, a substantially homogenous, or a heterogeneous culture of cells. Generally, a "population" may also be regarded as an "isolated" culture of cells.

As used herein, a "recombinant cell" is a host cell that comprises a recombinant polynucleotide.

"Sample" or "biological sample" as used herein means a biological material from a subject, including but is not limited to organ, tissue, exosome, blood, plasma, saliva, urine and other body fluid. A sample can be any source of material obtained from a subject.

"Signal 1" as used herein generally refers to the first biochemical signal passed from an activated DC to a T cell. Signal 1 is provided by an antigen expressed at the surface of the DC and is sensed by the T cell through the T cell receptor.

"Signal 2" as used herein generally refers to the second signal provided by DCs to T cells. Signal 2 is provided by "costimulatory" molecules on the activated DC, usually CD80 and/or CD86 (although there are other co-stimulatory molecules known), and is sensed by the T cell through the surface receptor CD28.

"Signal 3" as used herein generally refers to the signal generated from soluble proteins (usually cytokines) produced by the activated DC. These are sensed through receptors on the T lymphocyte. The 3^{rd} signal instructs the T cell as to which phenotypical or functional features they should acquire to best deal with the current threat.

By the term "specifically binds," as used herein, is meant a molecule, such as an antibody, which recognizes and binds to another molecule or feature, but does not substantially recognize or bind other molecules or features in a sample.

The terms "subject," "patient," "individual," and the like are used interchangeably herein, and refer to any animal, or cells thereof whether *in vitro* or *in situ,* amenable to the methods described herein. In certain non-limiting embodiments, the patient, subject or individual is a human.

The term "T cell" as used herein is defined as a thymus-derived cell that participates in a variety of cell-mediated immune reactions.

The term "T-helper" as used herein with reference to cells indicates a subgroup of lymphocytes (a type of white blood cell or leukocyte) including different cell types identifiable by a skilled person. In particular, T-helper cell according to the present disclosure include effector Th cells (such as Th1, Th2 and Th17). These Th cells secrete cytokines, proteins or peptides that stimulate or interact with other leukocytes.

"Th1 T cell" as used herein refers to a T cell that produces high levels of the cytokine IFN-γ and is thought to be highly effective against certain disease-causing microbes that live inside host cells, and cancer as well.

"Th17 T cell" as used herein refers to a T cell that produces high levels of the cytokines IL-17 and IL-22 and is thought to be highly effective against disease-causing microbes that live on mucousal surfaces.

"Therapeutically effective amount" is an amount of a compound of the invention, that when administered to a patient, ameliorates a symptom of the disease. The amount of a compound of the invention which constitutes a "therapeutically effective amount" will vary depending on the compound, the disease state and its severity, the age of the patient to be treated, and the like. The therapeutically effective amount can be determined routinely by one of ordinary skill in the art having regard to his own knowledge and to this disclosure.

The terms "treat," "treating," and "treatment," refer to therapeutic or preventative measures described herein. The methods of "treatment" employ administration to a subject, in need of such treatment, a composition of the present invention, for example, a subject afflicted a disease or disorder, or a subject who ultimately may acquire such a disease or disorder, in order to prevent, cure, delay, reduce the severity of, or ameliorate one or more symptoms of the disorder or recurring disorder, or in order to prolong the survival of a subject beyond that expected in the absence of such treatment.

The term "Toll like receptor", or "TLR" as used herein is defined as a class of proteins that play a role in the innate immune system. TLRs are single membrane-spanning, non-catalytic receptors that recognize structurally conserved molecules derived from microbes. TLRs activate immune cell responses upon binding to a ligand.

The term "Toll like receptor agonists", or "TLR agonists" as used herein is defined as a ligand that binds to the TLR to activate immune cell response.

The term "vaccine" as used herein is defined as a material used to provoke an immune response after administration of the material to an animal, preferably a mammal, and more preferably a human. Upon introduction into a subject, the vaccine is able to provoke an immune response including, but not limited to, the production of antibodies, cytokines and/or other cellular responses.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

### Description

The invention includes a preparation of DCs. In one embodiment, the DC preparations are greater than 90% pure. In another embodiment, the DC preparations are fully activated. For example, the DCs are activated with a cytokine and/or a Toll like receptor ligand, a state which is fully maintained by the cryopreservation technique of the invention. A benefit of the DC preparation of the invention is that the cells are efficiently cryopreserved from a single leukapheresis (patient collection) into an initial vaccine plus multiple "booster" doses (e.g., 10 or more) that can be thawed as needed at remote treatment locations without any specialized cell processing facilities or further required quality control testing.

As contemplated herein, the present invention provides a method for generating and cryopreserving DCs with superior functionality in producing stronger signals to T cells, and thus resulting in a more potent DC-based vaccine. By effectively cryopreserving such cells, samples can be stored and thawed for later use, thereby reducing the need for repeated pheresis and elutriation processes during vaccine production. Being able to freeze DCs and then thaw them out later is an advantage because it means that a single round of vaccine production can be divided into small parts, frozen away, and then administered one at a time to a patient over the course of weeks, months, or years to give "booster" vaccinations that strengthen immunity.

In one embodiment, the invention includes an FDA- approved injectable multi-dose antigen pulsed dendritic cell vaccine produced by collecting DCs in a single patient leukapheresis. The FDA- approved injectable multi-dose antigen pulsed dendritic cell vaccine comprises an initial immunizing dose and multiple "booster" doses. The FDA-approved injectable multi-dose antigen pulsed dendritic cell vaccine are cryopreserved and can be shipped to remote medical centers for serial administration to the patient with no special requirements at the administration site (e.g., FDA mandated QC/QA steps).

The present invention also relates to the cryopreservation of these activated DCs in a manner that retains their potency and functionality in presenting antigen as well as their production of various cytokines and chemokines after thawing, such that the cryopreserved and subsequently thawed activated DCs are as clinically effective as freshly harvested and activated DCs.

The present invention also relates to inducing tumor senescence and apoptosis in a cell by blocking one or more of HER-2 and HER-3 in combination with activating anti-HER-2 CD4 Th1 cells. Accordingly, the invention includes a combination and method for promoting an anti-oncodriver Th1 immune response with an oncodriver blockade for HER-2 in order to promote tumor senescent in HER-2 expressing breast cancers. In one embodiment, promoting an anti-oncodriver Th1 immune response comprises TNF-α and IFN-γ. In one embodiment, an oncodriver blockade for HER-2 includes any composition that blocks HER-2 including but is not limited to trastuzumab and pertuzumab.

In one embodiment, the invention includes compositions and methods for the combination of blocking one or more of HER-2 and HER-3 together with the addition of TNF-α and IFN-γ for inducing senescence of HER-2 expressing breast cancer. In one embodiment, the TNF-α and IFN-γ is secreted from CD4 Th1 cells.

In one embodiment, HER2 is required in the mechanism of TNF-α and IFN-γ inducing senescence and apoptosis in breast cancer cells.

In one embodiment, TNF-α and IFN-γ restores the sensitivity to trastuzumab and pertuzumab to breast cancer resistant cells. In one embodiment, the Th1 cytokines, IFN-γ and TNF-α, revert the resistance to the therapeutic agents that is affecting cancer patients widely.

### DC-Based Immunotherapy

DCs are derived from pluripotent monocytes that serve as antigen-presenting cells (APCs). DCs are ubiquitous in peripheral tissues, where they are prepared to capture antigens. Upon antigen capture, DCs process the antigen into small peptides and move towards secondary lymphoid organs. It is within the lymphoid organs that DCs present antigen peptides to naive T cells, thereby initiating a cascade of signals that polarizes T cell differentiation. Upon exposure, DCs present antigen molecules bound to either MHC class I or class II binding peptides and activate CD8⁺ or CD4⁺ T cells, respectively (Steinman, 1991, Annu. Rev. Immunol. 9:271-296; Banchereau et al., 1998, Nature392,245-252; Steinman, et al., 2007, Nature 449:419-426; Ginhoux et al., 2007, J. Exp. Med. 204:3133-3146; Banerjee et al., 2006, Blood 108:2655-2661; Sallusto et al., 1999, J. Exp. Med. 189:611-614; Reid et al., 2000, Curr. Opin. Immuno1.12:114-121; Bykovskaia et al., 1999, J. Leukoc. Biol. 66:659-666; Clark et al., 2000, Microbes Infect. 2:257-272).

DCs are responsible for the induction, coordination and regulation of the adaptive immune response and also serve to orchestrate communication between effectors of the innate arm and the adaptive arm of the immune system. These features have made DCs strong candidates for immunotherapy. DCs have a unique capacity to sample the environment through macropinocytosis and receptor-mediated endocytosis (Gerner et al., 2008, J. Immunol.181:155-164; Stoitzner et al., 2008, Cancer Immunol. Immunother 57:1665-1673; Lanzevecchia A., 1996, Curr. Opin. Immunol.8:348-354; Delamarre et al., 2005, Science, 307(5715):1630-1634).

DCs also require maturation signals to enhance their antigen-presenting capacity. DCs upregulate the expression of surface molecules, such as CD80 and CD86 (also known as second signal molecules) by providing additional maturation signals, such as TNF-α, CD40L or calcium signaling agents (Czerniecki et al., 1997,. J. Immuno1.159:3823-3837; Bedrosian et al. 2000, J. Immunother. 23:311-320; Mailliard et al., 2004, Cancer Res.64,5934-5937; Brossart et al., 1998, Blood 92:4238-4247; Jin et al., 2004, Hum. Immunol. 65:93-103). It has been established that a mixture of cytokines, including TNF-α, IL-1β, IL-6 and prostaglandin E2 (PGE2), have the ability to mature DC (Jonuleit, et al., 2000, Arch. Derm. Res. 292:325-332). DCs can also be matured with calcium ionophore prior to being pulsed with antigen.

In addition to pathogen-recognition receptors, such as PKR and MDA-5 (Kalali et al., 2008, J. Immunol. 181:2694-2704; Nallagatla et al., 2008, RNA Biol. 5(3):140-144), DCs also contain a series of receptors, known as Toll-like receptors (TLRs), that are also capable of sensing danger from pathogens. When these TLRs are triggered, a series of activational changes are induced in DCs, which lead to maturation and signaling of T cells (Boullart et al. 2008, Cancer Immunol. Immunother. 57(11):1589-1597; Kaisho et al., 2003, Curr. Mol. Med. 3(4):373-385; Pulendran et al., 2001, Science 293(5528):253-256; Napolitani et al., 2005, Nat. Immunol. 6(8):769-776). DCs can activate and extend the various arms of the cell-mediated response, such as natural killer γ-δ T and α-β T cells and, once activated, DCs retain their immunizing capacity (Steinman, 1991, Annu. Rev. Immunol. 9:271-296; Banchereau et al., 1998, Nature 392:245-252; Reid et al., 2000, Curr. Opin. Immunol. 12:114-121; Bykovskaia et al., 1999, J. Leukoc. Biol.66:659-666; Clark et al., 2000, Microbes Infect. 2:257-272).

The present invention includes mature, antigen loaded DCs activated by Toll-like receptor agonists that induce clinically effective immune responses, preferably when used earlier in the disease process. The DCs of the present invention produce desirable levels of cytokines and chemokines, and further have the capacity to induce apoptosis of tumor cells.

In one embodiment, the invention provides a method of large scale production of antigen pulsed dendritic cell vaccine. In one embodiment, the method comprises rapidly maturing dendritic cells, cryopreserving the dendritic cells, and thawing the cryopreserved cells wherein the thawed dendritic cells produce an effective amount of at least one cytokine to generate a T cell response.

In one embodiment, the maturation of dendritic cells comprise contacting the cells with IFN-gamma and LPS.

In one embodiment, the thawed cells maintain DC1 phenotype to drive a Th1 polarized immune response.

In one embodiment, the thawed cells maintain the ability to primarily sensitize T cells.

### Generation of a loaded (pulsed) immune cell

The present invention includes a cell that has been exposed or otherwise "pulsed" with an antigen. For example, an APC, such as a DC, may become Ag-loaded *in vitro,* e.g., by culture *ex vivo* in the presence of an antigen, or *in vivo* by exposure to an antigen.

A person skilled in the art would also readily understand that an APC can be "pulsed" in a manner that exposes the APC to an antigen for a time sufficient to promote presentation of that antigen on the surface of the APC. For example, an APC can be exposed to an antigen in the form of small peptide fragments, known as antigenic peptides, which are "pulsed" directly onto the outside of the APCs (Mehta-Damani *et al.,* 1994); or APCs can be incubated with whole proteins or protein particles which are then ingested by the APCs. These whole proteins are digested into small peptide fragments by the APC and eventually carried to and presented on the APC surface (Cohen *et al.,* 1994). Antigen in peptide form may be exposed to the cell by standard "pulsing" techniques described herein.

Without wishing to be bound by any particular theory, the antigen in the form of a foreign or an autoantigen is processed by the APC of the invention in order to retain the immunogenic form of the antigen. The immunogenic form of the antigen implies processing of the antigen through fragmentation to produce a form of the antigen that can be recognized by and stimulate immune cells, for example T cells. Preferably, such a foreign or an autoantigen is a protein which is processed into a peptide by the APC. The relevant peptide which is produced by the APC may be extracted and purified for use as an immunogenic composition. Peptides processed by the APC may also be used to induce tolerance to the proteins processed by the APC.

The antigen-loaded APC, otherwise known as a "pulsed APC" of the invention, is produced by exposure of the APC to an antigen either *in vitro* or *in vivo.* In the case where the APC is pulsed *in vitro,* the APC can be plated on a culture dish and exposed to an antigen in a sufficient amount and for a sufficient period of time to allow the antigen to bind to the APC. The amount and time necessary to achieve binding of the antigen to the APC may be determined by using methods known in the art or otherwise disclosed herein. Other methods known to those of skill in the art, for example immunoassays or binding assays, may be used to detect the presence of antigen on the APC following exposure to the antigen.

In a further embodiment of the invention, the APC may be transfected with a vector which allows for the expression of a specific protein by the APC. The protein which is expressed by the APC may then be processed and presented on the cell surface. The transfected APC may then be used as an immunogenic composition to produce an immune response to the protein encoded by the vector.

As discussed elsewhere herein, vectors may be prepared to include a specific polynucleotide which encodes and expresses a protein to which an immunogenic response is desired. Preferably, retroviral vectors are used to infect the cells. More preferably, adenoviral vectors are used to infect the cells.

In another embodiment, a vector may be targeted to an APC by modifying the viral vector to encode a protein or portions thereof that is recognized by a receptor on the APC, whereby occupation of the APC receptor by the vector will initiate endocytosis of the vector, allowing for processing and presentation of the antigen encoded by the nucleic acid of the viral vector. The nucleic acid which is delivered by the virus may be native to the virus, which when expressed on the APC encodes viral proteins which are then processed and presented on the MHC receptor of the APC.

As contemplated herein, various methods can be used for transfecting a polynucleotide into a host cell. The methods include, but are not limited to, calcium phosphate precipitation, lipofection, particle bombardment, microinjection, electroporation, colloidal dispersion systems (i.e. macromolecule complexes, nanocapsules, microspheres, beads, and lipid-based systems including oil-in-water emulsions, micelles, mixed micelles, and liposomes). These methods are understood in the art and are described in published literature so as to enable one skilled in the art to perform these methods.

In another embodiment, a polynucleotide encoding an antigen can be cloned into an expression vector and the vector can be introduced into an APC to otherwise generate a loaded APC. Various types of vectors and methods of introducing nucleic acids into a cell are discussed in the available published literature. For example, the expression vector can be transferred into a host cell by physical, chemical or biological means. See, for example, Sambrook et al. (2001, Molecular Cloning: A Laboratory Manual, Cold Spring Harbor Laboratory, New York), and in Ausubel et al. (1997, Current Protocols in Molecular Biology, John Wiley & Sons, New York). It is readily understood that the introduction of the expression vector comprising a polynucleotide encoding an antigen yields a pulsed cell.

The present invention includes various methods for pulsing APCs including, but not limited to, loading APCs with whole antigen in the form of a protein, cDNA or mRNA. However, the invention should not be construed to be limited to the specific form of the antigen used for pulsing the APC. Rather, the invention encompasses other methods known in the art for generating an antigen loaded APC. Preferably, the APC is tranfected with mRNA encoding a defined antigen. mRNA corresponding to a gene product whose sequence is known can be rapidly generated *in vitro* using appropriate primers and reverse transcriptase-polymerase chain reaction (RT-PCR) coupled with transcription reactions. Transfection of an APC with an mRNA provides an advantage over other antigen-loading techniques for generating a pulsed APC. For example, the ability to amplify RNA from a microscopic amount of tissue, i.e. tumor tissue, extends the use of the APC for vaccination to a large number of patients.

For an antigenic composition to be useful as a vaccine, the antigenic composition must induce an immune response to the antigen in a cell, tissue or mammal (e.g., a human). As used herein, an "immunological composition" may comprise an antigen (e.g., a peptide or polypeptide), a nucleic acid encoding an antigen (e.g., an antigen expression vector), or a cell expressing or presenting an antigen or cellular component. In particular embodiments the antigenic composition comprises or encodes all or part of any antigen described herein, or an immunologically functional equivalent thereof. In other embodiments, the antigenic composition is in a mixture that comprises an additional immunostimulatory agent or nucleic acids encoding such an agent. Immunostimulatory agents include but are not limited to an additional antigen, an immunomodulator, an antigen presenting cell or an adjuvant. In other embodiments, one or more of the additional agent(s) is covalently bonded to the antigen or an immunostimulatory agent, in any combination. In certain embodiments, the antigenic composition is conjugated to or comprises an HLA anchor motif amino acids.

A vaccine, as contemplated herein, may vary in its composition of nucleic acid and/or cellular components. In a non-limiting example, a nucleic encoding an antigen might also be formulated with an adjuvant. Of course, it will be understood that various compositions described herein may further comprise additional components. For example, one or more vaccine components may be comprised in a lipid or liposome. In another non-limiting example, a vaccine may comprise one or more adjuvants. A vaccine of the present invention, and its various components, may be prepared and/or administered by any method disclosed herein or as would be known to one of ordinary skill in the art, in light of the present disclosure.

It is understood that an antigenic composition of the present invention may be made by a method that is well known in the art, including but not limited to chemical synthesis by solid phase synthesis and purification away from the other products of the chemical reactions by HPLC, or production by the expression of a nucleic acid sequence (e.g., a DNA sequence) encoding a peptide or polypeptide comprising an antigen of the present invention in an *in vitro* translation system or in a living cell. In addition, an antigenic composition can comprise a cellular component isolated from a biological sample. The antigenic composition isolated and extensively dialyzed to remove one or more undesired small molecular weight molecules and/or lyophilized for more ready formulation into a desired vehicle. It is further understood that additional amino acids, mutations, chemical modification and such like, if any, that are made in a vaccine component will preferably not substantially interfere with the antibody recognition of the epitopic sequence.

A peptide or polypeptide corresponding to one or more antigenic determinants of the present invention should generally be at least five or six amino acid residues in length, and may contain up to about 10, about 15, about 20, about 25, about 30, about 35, about 40, about 45 or about 50 residues or so. A peptide sequence may be synthesized by methods known to those of ordinary skill in the art, such as, for example, peptide synthesis using automated peptide synthesis machines, such as those available from Applied Biosystems, Inc., Foster City, CA (Foster City, CA).

Longer peptides or polypeptides also may be prepared, e.g., by recombinant means. In certain embodiments, a nucleic acid encoding an antigenic composition and/or a component described herein may be used, for example, to produce an antigenic composition *in vitro* or *in vivo* for the various compositions and methods of the present invention. For example, in certain embodiments, a nucleic acid encoding an antigen is comprised in, for example, a vector in a recombinant cell. The nucleic acid may be expressed to produce a peptide or polypeptide comprising an antigenic sequence. The peptide or polypeptide may be secreted from the cell, or comprised as part of or within the cell.

In certain embodiments, an immune response may be promoted by transfecting or inoculating a mammal with a nucleic acid encoding an antigen. One or more cells comprised within a target mammal then expresses the sequences encoded by the nucleic acid after administration of the nucleic acid to the mammal. A vaccine may also be in the form, for example, of a nucleic acid (e.g., a cDNA or an RNA) encoding all or part of the peptide or polypeptide sequence of an antigen. Expression *in vivo* by the nucleic acid may be, for example, by a plasmid type vector, a viral vector, or a viral/plasmid construct vector.

In another embodiment, the nucleic acid comprises a coding region that encodes all or part of the sequences encoding an appropriate antigen, or an immunologically functional equivalent thereof. Of course, the nucleic acid may comprise and/or encode additional sequences, including but not limited to those comprising one or more immunomodulators or adjuvants.

### Antigens

As contemplated herein, the present invention may include use of any antigen suitable for loading into an APC to elicit an immune response. In one embodiment, tumor antigens may be used. Tumor antigens can be divided into two broad categories: *shared* tumor antigens; and *unique* tumor antigens. Shared antigens are expressed by many tumors, while unique tumor antigens can result from mutations induced through physical or chemical carcinogens, and are therefore expressed only by individual tumors. In certain embodiments, shared tumor antigens are loaded into the DCs of the present invention. In other embodiments, unique tumor antigens are loaded into the DCs of the present invention.

In the context of the present invention, "tumor antigen" refer to antigens that are common to specific hyperproliferative disorders. In certain aspects, the hyperproliferative disorder antigens of the present invention are derived from cancers, including but not limited to, primary or metastatic melanoma, thymoma, lymphoma, sarcoma, lung cancer, liver cancer, non-Hodgkin's lymphoma, Hodgkins lymphoma, leukemias, uterine cancer, cervical cancer, bladder cancer, kidney cancer and adenocarcinomas such as breast cancer, prostate cancer, ovarian cancer, pancreatic cancer, and the like.

Malignant tumors express a number of proteins that can serve as target antigens for an immune attack. These molecules include, but are not limited to, tissue-specific antigens such as MART-1, tyrosinase and GP 100 in melanoma, and prostatic acid phosphatase (PAP) and prostate-specific antigen (PSA) in prostate cancer. Other target molecules belong to the group of transformation-related molecules, such as the oncogene HER-2/Neu/ErbB-2. Yet another group of target antigens are onco-fetal antigens, such as carcinoembryonic antigen (CEA). In B cell lymphoma, the tumor-specific idiotype immunoglobulin constitutes a truly tumor-specific immunoglobulin antigen that is unique to the individual tumor. B cell differentiation antigens, such as CD19, CD20 and CD37, are other candidates for target antigens in B cell lymphoma. Some of these antigens (CEA, HER-2, CD 19, CD20, idiotype) have been used as targets for passive immunotherapy with monoclonal antibodies with limited success.

The tumor antigen and the antigenic cancer epitopes thereof may be purified and isolated from natural sources such as from primary clinical isolates, cell lines and the like. The cancer peptides and their antigenic epitopes may also be obtained by chemical synthesis or by recombinant DNA techniques known in the arts. Techniques for chemical synthesis are described in Steward et al. (1969); Bodansky et al. (1976); Meienhofer (1983); and Schroder et al. (1965). Furthermore, as described in Renkvist et al. (2001), there are numerous antigens known in the art. Although analogs or artificially modified epitopes are not specifically described, a skilled artisan recognizes how to obtain or generate them by standard means in the art. Other antigens, identified by antibodies and as detected by the Serex technology (see Sahin et al. (1997) and Chen et al. (2000)), are identified in the database of the Ludwig Institute for Cancer Research.

In yet another embodiment, the present invention may include microbial antigens for presentation by the APCs. As contemplated herein, microbial antigens may be viral, bacterial, or fungal in origin. Examples of infectious virus include: Retroviridae (e.g. human immunodeficiency viruses, such as HIV-1 (also referred to as HTLV-III, LAV or HTLV-III/LAV, or HIV-III; and other isolates, such as HIV-LP; Picornaviridae (e.g. polio viruses, hepatitis A virus; enteroviruses, human coxsackie viruses, rhinoviruses, echoviruses); Calciviridae (e.g. strains that cause gastroenteritis); Togaviridae (e.g. equine encephalitis viruses, rubella viruses); Flaviridae (e.g. dengue viruses, encephalitis viruses, yellow fever viruses); Coronaviridae (e.g. coronaviruses); Rhabdoviridae (e.g. vesicular stomatitis viruses, rabies viruses); Filoviridae (e.g. ebola viruses); Paramyxoviridae (e.g. parainfluenza viruses, mumps virus, measles virus, respiratory syncytial virus); Orthomyxoviridae (e.g. influenza viruses); Bungaviridae (e.g. Hantaan viruses, bunga viruses, phleboviruses and Nairo viruses); Arena viridae (hemorrhagic fever viruses); Reoviridae (e.g. reoviruses, orbiviruses and rotaviruses); Birnaviridae; Hepadnaviridae (Hepatitis B virus); Parvovirida (parvoviruses); Papovaviridae (papilloma viruses, polyoma viruses); Adenoviridae (most adenoviruses); Herpesviridae (herpes simplex virus (HSV) 1 and 2, varicella zoster virus, cytomegalovirus (CMV), herpes virus); Poxviridae (variola viruses, vaccinia viruses, pox viruses); and Iridoviridae (e.g. African swine fever virus); and unclassified viruses (e.g. the etiological agents of Spongiform encephalopathies, the agent of delta hepatitis (thought to be a defective satellite of hepatitis B virus), the agents of non-A, non-B hepatitis (class 1=intemally transmitted; class 2=parenterally transmitted (i.e. Hepatitis C); Norwalk and related viruses, and astroviruses).

Examples of infectious bacteria include: Helicobacter pyloris, Borelia burgdorferi, Legionella pneumophilia, Mycobacteria sps (e.g. M. tuberculosis, M. avium, M. intracellulare, M. kansasii, M. gordonae), Staphylococcus aureus, Neisseria gonorrhoeae, Neisseria meningitidis, Listeria monocytogenes, Streptococcus pyogenes (Group A Streptococcus), Streptococcus agalactiae (Group B Streptococcus), Streptococcus (viridans group), Streptococcus faecalis, Streptococcus bovis, Streptococcus (anaerobic sps.), Streptococcus pneumoniae, pathogenic Campylobacter sp., Enterococcus sp., Haemophilus influenzae, Bacillus anthracis, corynebacterium diphtheriae, corynebacterium sp., Erysipelothrix rhusiopathiae, Clostridium perfringens, Clostridium tetani, Enterobacter aerogenes, Klebsiella pneumoniae, Pasturella multocida, Bacteroides sp., Fusobacterium nucleatum, Streptobacillus moniliformis, Treponema Treponema pertenue, Leptospira, and Actinomyces israelli.

Examples of infectious fungi include: Cryptococcus neoformans, Histoplasma capsulatum, Coccidioides immitis, Blastomyces dermatitidis, Chlamydia trachomatis, Candida albicans. Other infectious organisms (i.e., protists) including: Plasmodium falciparum and Toxoplasma gondii.

### Activation of DCs

While traditional DC-based vaccines (that have previously dominated clinical trials) comprise of matured DCs generated from using a cytokine cocktail mixture including combinations of TNF, IL-6, PGE2 and IL-1β, which ultimately stimulate aseptic inflammation, the present invention instead utilizes TLR agonists to mature the DCs and stimulate production of signal.

According to an aspect of the present invention, the stimulation of DCs with a combination of TLR ligands leads to the production of increased amounts of IL-12. Further, activation of DCs with a combination of TLR agonists can yield a more pronounced CD4 and CD8 T-cell response (Warger et al., 2006, Blood 108:544-550). Thus, the DCs of the present invention can secrete Th1 driving cytokines, such as IL-12, by exposure to these ligands that trigger TLRs. For example, the addition of poly(I:C), a TLR3 agonist to IL-1β, TNF-α, and IFN-γ, can generate a potent type-1 polarized DC, characterized by robust levels of IL-12 production (Heifler et al., 1996, Eur. J. Immunol. 26:659-668). In certain embodiments, antigen can be loaded into the DC prior to TLR agonist exposure. In other embodiments, antigen can be loaded into the DC subsequent to TLR agonist exposure.

According to an aspect of the present invention, the injectable multi-dose antigen pulsed dendritic cell vaccine is produced by collecting DCs in a single patient leukapheresis whereby the cells are activated with biomolecules that simulate bacterial infection (e.g., LPS). This unique activation method endows the DCs with qualities not found in DCs that are matured with a cytokine cocktail of TNF, IL-6, PGE2 and IL-1β (the "traditional maturation"), which also simulates aseptic inflammation (Lombardi et al., 2009, J. Immunol. 182:3372-3379).

In one embodiment, the DCs of the present invention can be activated with the combination of the TLR4 agonist, bacterial lipopolysaccharide (LPS), the TLR7/8 agonist, resimiquod (R848) and/or IFN-γ (Amati et al., 2006, Curr. Pharm. Des 12:4247-4254). By activating DCs with a TLR4 agonist and bacterial LPS, DCs are generated that are at least virtually identical (in phenotype) to DC1s generated via traditional maturation methods. These DCs have a high expression of surface molecules, including CD83, CD80, CD86 and HLA-DR. In other embodiments, TLR2 agonists, such as lipotechoic acid (LTA), TLR3 agonists, such as poly(I:C), and/or other TLR4 agonists, such as MPL, may be used. As contemplated herein, any TLR agonist, or combination of TLR agonists, can be used to active DCs, provided such ligands stimulate the production of cytokine and chemokine signals by the activated DCs. Many other TLR agonists are known in the art and can be found in the published literature for use with the present invention.

Even though there are similarities in phenotype between DCs and traditionally matured DCs, the DCs of the present invention display many marked advantages.

### Cryopreservation

After culture initiation and activation, the cells are harvested and the vaccine is cryopreserved. For example, peripheral blood monocytes are obtained by leukapheresis. The cells are cultured in serum free medium with GM-CSF and IL-4 for a period of time followed by pulsing the cells with a desired antigen. Following pulsing the cells with a desired antigen, the antigen pulsed dendritic cells are incubated with IFN-γ followed by a TLR agonist (e.g., LPS). The activated antigen pulsed dendritic cell is harvested and cryopreserved in a freezing medium and stored in liquid nitrogen. In one embodiment the freezing medium comprises 55% plasmalyte, 40% human serum albumin, and 5% DMSO.

The cryopreservation aspect of the invention allows for the generation of an FDA- approved injectable multi-dose antigen pulsed dendritic cell vaccine. An advantage of the invention is that the multi-dose antigen pulsed dendritic cells retain their ability to produce signals critical to T cell function after thawing. As contemplated herein, the present invention includes a variety of cryopreservation techniques and cryomedia, as would be understood by those skilled in the art. For example, in certain embodiments, the freezing medium comprises 55% plasmalyte, 40% human serum albumin, and 5% DMSO. Accordingly, the invention provides the ability to produce the multi-dose antigen pulsed dendritic cell vaccine of the invention at a centralized area comprising of an initial immunizing dose and multiple "booster" doses. Therefore the multi-dose antigen pulsed dendritic cell vaccine can be shipped to remote medical centers for serial administration to the patient with no special FDA quality control/quality assurance requirements at the administration site.

In one embodiment, the dendritic cell vaccine of the invention is cryopreserved in aliquots for multiple doses. For example, the cells are cryopreserved at a concentration of 30x10⁶ cells/mL. For example, a bag of freezing medium containing a volume equal to the cell volume is prepared. Working rapidly, the freezing medium is added to the cell bag and the cells are transferred to labeled cryovials. In one embodiment, the vials are frozen using a rate controlled freezer. For example, cryovials are frozen using an automated rate controlled freezer at 1°C/min and stored in vapor phase nitrogen.

In one embodiment, the vials are frozen using a rate controlled freezer. The vials are placed in a freezing chamber and liquid nitrogen enters the chamber through an electronic solenoid valve. Since vaporization is almost instantaneous, controlling the rate at which liquid nitrogen enters the chamber directly controls the rate at which heat is absorbed and removed from the freezing chamber and its contents.

As contemplated herein, the present invention includes a variety of cryopreservation techniques and freezing medium, as would be understood by those skilled in the art. For example, in certain embodiments, the freezing medium for cultured cells can include about 55% plasmalyte, about 40% human serum albumin, and about 5% DMSO. In other embodiments, the cryomedia can be serum-free. In certain embodiments, controlled rate freezing may be used, while other embodiments can include use of insulated containers in which vials of cells mixed with freezing medium are placed in the freezer, such as at temperatures ranging from about -70°C to -80°C. The present invention provides a method to preserve activated DCs in such a manner so as to further facilitate clinical application of such cells, and to reduce the need for extensive and repeated pherisis and elutriation steps. As contemplated herein, cryopreservation techniques may be used for both small-scale and large-scale batches.

When considering the extensive utility for activated DC, the ability to provide a steady supply of cryopreserved activated DC represents a significant advantage that can facilitate various therapeutic uses of such cells. For example, a large-scale culture of activated DC may be cryopreserved in aliquots of the appropriate size comprising an initial immunizing dose and multiple "booster" doses, according to the methods of the present invention, such that individual doses of cells can later be used in any particular immunotherapeutic protocol. In certain embodiments, activated DCs can be cryopreserved for 2-24 weeks at temperatures of approximately -70°C or lower. At lower temperatures, such as at about -120°C or lower, activated DCs can be cryopreserved for at least a year or longer.

In one exemplary embodiment, the DCs are suspended in human serum and approximately 5% DMSO (v/v). Alternatively, other serum types, such as fetal calf serum, may be used. The suspended cells can be aliquoted into smaller samples, such as in 1.8 ml vials, and stored at approximately -70°C or lower. In other embodiments, the freezing medium may include about 20% serum and about 10% DMSO, and suspended cells can be stored at about -180°C. Still further embodiments may include medium containing about 55% plasmalyte, and about 5% DMSO. Other exemplary freezing media may include about 12% DMSO and about 25-40% serum.

While the present invention as described herein may include specific concentrations of serum, it should be understood by those skilled in the art that the exact amount of serum in the freezing medium may vary, and in some embodiments may be entirely absent, but will generally be within the range of about 1% to 30%. Of course, any concentration of serum that results in a cell viability of around 50% and/or a cell recovery of around 50% may be used in any DC composition of the present invention, as well as with any cryopreservation method as described herein. Preferably, cell viability and recovery of at least 60%, more preferably at least about 70%, or even 80% is desired when recovering cryopreserved cells in the selected freezing medium.

Similarly, while the present invention as described herein may include specific concentrations of DMSO, those skilled in the art should recognize that DMSO may be entirely absent in some embodiments, while in other embodiments, concentrations from about 5% to as high as about 20% may be used in the freezing medium and included within the cryopreservation methods described herein. Generally, lower concentrations of DMSO are preferred, such as between about 5% to about 10%. However, any concentration of DMSO that results, after thawing, in cell viability of at least 50% and a cell recovery of at least 50%, and preferably a cell viability and recovery of at least 60%, more preferably about 70%, more preferably about 80% and even more preferably about 90% and higher, may be used.

While the present invention as described herein may include reference to a rate controlled freezing, it should be understood by those skilled in the art that methods of freezing in a rate controlled or non-rate controlled manner can routinely be employed. It should also be understood by those skilled in the art that the various cryopreservation media as described herein may either include serum or may be serum free. Examples of serum free media can include XVIVO 10, XVIVO 15, XVIVO 20, StemPro, as well as any commercially available serum free media. When utilizing a serum-free freezing medium, the cryopreservation methods of the present invention are generally free of infectious agents, antibodies and foreign proteins, which may be antigenic, and any other foreign molecule that may typically be found in serum-based freezing medium.

Cryopreservation of antigen loaded, active DCs can occur at any point after activation of the cells with a TLR agonist. In one embodiment, the activated DCs are cryopreserved approximately 6-8 hr after exposure to the TLR agonist. Preferably, the time point chosen to cryopreserve the activated cells should be based on the maximization of signal production of the cells, particularly IL-12 production.

The present invention provides compositions and methods for producing large scale dendritic cell vaccines. In one embodiment, the large scale production of dendritic cell vaccines allows for the production of an FDA-approved injectable multi-dose antigen pulsed dendritic cell vaccine for the personalized treatment and prevention of cancer or other disorders. In one embodiment, the invention provides compositions and methods for producing large scale of antigen pulsed type I polarized dendritic cell vaccine (DC1).

In one embodiment, the invention provides a method to cryopreserve dendritic cells that are in an antigen-loaded, pre-activated state in a large scale that is "syringe-ready", i.e. suitable for immediate injection into the patient without the necessity of any further cell processing that would require (e.g., by FDA mandate) additional facilities and quality control/assurance steps.

In one embodiment, the invention provides a method to efficiently produce in a large scale injectable multi-dose antigen pulsed dendritic cell vaccine, preferably injectable multi-dose antigen pulsed type I polarized dendritic cell vaccine that exhibit maximal efficacy.

### Packaging of Compositions or Kit Components

Suitable containers for compositions of the invention (or kit components) include vials, syringes (e.g. disposable syringes), etc. These containers should be sterile.

Where a composition/component is located in a vial, the vial is preferably made of a glass or plastic material. The vial is preferably sterilized before the composition is added to it. To avoid problems with latex-sensitive patients, vials are preferably sealed with a latex-free stopper, and the absence of latex in all packaging material is preferred. The vial may include a single dose of vaccine, or it may include more than one dose (a "multidose" vial) e.g. 10 doses. Preferred vials are made of colorless glass.

A vial can have a cap (e.g. a Luer lock) adapted such that a pre-filled syringe can be inserted into the cap, the contents of the syringe can be expelled into the vial, and the contents of the vial can be removed back into the syringe. After removal of the syringe from the vial, a needle can then be attached and the composition can be administered to a patient. The cap is preferably located inside a seal or cover, such that the seal or cover has to be removed before the cap can be accessed. A vial may have a cap that permits aseptic removal of its contents, particularly for multidose vials.

Where a composition/component is packaged into a syringe, the syringe may have a needle attached to it. If a needle is not attached, a separate needle may be supplied with the syringe for assembly and use. Such a needle may be sheathed. Safety needles are preferred. 1-inch 23-gauge, 1-inch 25-gauge and 5/8-inch 25-gauge needles are typical. Syringes may be provided with peel-off labels on which the lot number, influenza season and expiration date of the contents may be printed, to facilitate record keeping. The plunger in the syringe preferably has a stopper to prevent the plunger from being accidentally removed during aspiration. The syringes may have a latex rubber cap and/or plunger. Disposable syringes contain a single dose of vaccine. The syringe will generally have a tip cap to seal the tip prior to attachment of a needle, and the tip cap is preferably made of a butyl rubber. If the syringe and needle are packaged separately then the needle is preferably fitted with a butyl rubber shield.

Containers may be marked to show a half-dose volume e.g. to facilitate delivery to children. For instance, a syringe containing a 0.5 ml dose may have a mark showing a 0.25 ml volume.

Where a glass container (e.g. a syringe or a vial) is used, then it is preferred to use a container made from a borosilicate glass rather than from a soda lime glass.

A kit or composition may be packaged (e.g. in the same box) with a leaflet including details of the vaccine e.g. instructions for administration, details of the antigens within the vaccine, etc. The instructions may also contain warnings e.g. to keep a solution of adrenaline readily available in case of anaphylactic reaction following vaccination, etc.

### Methods for Treating a Disease

The present invention also encompasses methods of treatment and/or prevention of a disease caused by pathogenic microorganisms, autoimmune disorder and/or a hyperproliferative disease.

Diseases that may be treated or prevented by use of the present invention include diseases caused by viruses, bacteria, yeast, parasites, protozoa, cancer cells and the like. The pharmaceutical composition of the present invention may be used as a generalized immune enhancer (DC activating composition or system) and as such has utility in treating diseases. Exemplary diseases that can be treated and/or prevented utilizing the pharmaceutical composition of the present invention include, but are not limited to infections of viral etiology such as HIV, influenza, Herpes, viral hepatitis, Epstein Bar, polio, viral encephalitis, measles, chicken pox, Papilloma virus etc.; or infections of bacterial etiology such as pneumonia, tuberculosis, syphilis, etc.; or infections of parasitic etiology such as malaria, trypanosomiasis, leishmaniasis, trichomoniasis, amoebiasis, etc.

Preneoplastic or hyperplastic states that may be treated or prevented using the pharmaceutical composition of the present invention (transduced DCs, expression vector, expression construct, etc.) of the present invention include but are not limited to preneoplastic or hyperplastic states such as colon polyps, Crohn's disease, ulcerative colitis, breast lesions and the like.

Cancers that may be treated using the composition of the present invention of the present invention include, but are not limited to primary or metastatic melanoma, adenocarcinoma, squamous cell carcinoma, adenosquamous cell carcinoma, thymoma, lymphoma, sarcoma, lung cancer, liver cancer, non-Hodgkin's lymphoma, Hodgkin's lymphoma, leukemias, uterine cancer, breast cancer, prostate cancer, ovarian cancer, pancreatic cancer, colon cancer, multiple myeloma, neuroblastoma, NPC, bladder cancer, cervical cancer and the like.

Other hyperproliferative diseases that may be treated using DC activation system of the present invention include, but are not limited to rheumatoid arthritis, inflammatory bowel disease, osteoarthritis, leiomyomas, adenomas, lipomas, hemangiomas, fibromas, vascular occlusion, restenosis, atherosclerosis, pre-neoplastic lesions (such as adenomatous hyperplasia and prostatic intraepithelial neoplasia), carcinoma in situ, oral hairy leukoplakia, or psoriasis.

Autoimmune disorders that may be treated using the composition of the present invention include, but are not limited to, AIDS, Addison's disease, adult respiratory distress syndrome, allergies, anemia, asthma, atherosclerosis, bronchitis, cholecystitis, Crohn's disease, ulcerative colitis, atopic dermatitis, dermatomyositis, diabetes mellitus, emphysema, erythema nodosum, atrophic gastritis, glomerulonephritis, gout, Graves' disease, hypereosinophilia, irritable bowel syndrome, lupus erythematosus, multiple sclerosis, myasthenia gravis, myocardial or pericardial inflammation, osteoarthritis, osteoporosis, pancreatitis, polymyositis, rheumatoid arthritis, scleroderma, Sjogren's syndrome, and autoimmune thyroiditis; complications of cancer, hemodialysis, and extracorporeal circulation; viral, bacterial, fungal, parasitic, protozoal, and helminthic infections; and trauma.

In the method of treatment, the administration of the composition of the invention may be for either "prophylactic" or "therapeutic" purpose. When provided prophylactically, the composition of the present invention is provided in advance of any symptom, although in particular embodiments the vaccine is provided following the onset of one or more symptoms to prevent further symptoms from developing or to prevent present symptoms from becoming worse. The prophylactic administration of composition serves to prevent or ameliorate any subsequent infection or disease. When provided therapeutically, the pharmaceutical composition is provided at or after the onset of a symptom of infection or disease. Thus, the present invention may be provided either prior to the anticipated exposure to a disease-causing agent or disease state or after the initiation of the infection or disease.

An effective amount of the composition would be the amount that achieves this selected result of enhancing the immune response, and such an amount could be determined as a matter of routine by a person skilled in the art. For example, an effective amount of for treating an immune system deficiency against cancer or pathogen could be that amount necessary to cause activation of the immune system, resulting in the development of an antigen specific immune response upon exposure to antigen. The term is also synonymous with "sufficient amount."

The effective amount for any particular application can vary depending on such factors as the disease or condition being treated, the particular composition being administered, the size of the subject, and/or the severity of the disease or condition. One of ordinary skill in the art can empirically determine the effective amount of a particular composition of the present invention without necessitating undue experimentation.

### Therapeutic applications

The present invention includes the generation of an antigen loaded, activated APC that produces significant levels of cytokines and chemokines when thawed from cryopreservation, where the antigen loaded and activated APC is used in immunotherapy for a mammal, preferably a human. The response to an antigen presented by an APC may be measured by monitoring the induction of a cytolytic T-cell response, a helper T-cell response, and/or antibody response to the antigen using methods well known in the art.

The present invention includes a method of enhancing the immune response in a mammal comprising the steps of: generating immature DCs from monocytes obtained from a mammal (e.g., a patient); pulsing the immature DCs with a composition comprising an antigenic composition; activating the antigen loaded DCs with at least one TLR agonist; cryopreserving the activated, antigen loaded DCs; thawing the activated, antigen loaded DCs and then administering the activated, antigen loaded DCs to a mammal in need thereof. The composition includes at least an antigen, and may further be a vaccine for *ex vivo* immunization and/or *in vivo* therapy in a mammal. Preferably, the mammal is a human.

*Ex vivo* procedures are well known in the art and are discussed more fully below. Briefly, cells are isolated from a mammal (preferably a human). The cells can be administered to a mammalian recipient to provide a therapeutic benefit. The mammalian recipient may be a human and the cells can be autologous with respect to the recipient. Alternatively, the cells can be allogeneic, syngeneic or xenogeneic with respect to the recipient.

In one embodiment, peripheral blood monocytes are obtained from a patient by combined leukapheresis and elutriation. The monocytes can be cultured in SFM with GM-CSF and IL-4 overnight. The next day, immature DCs can be pulsed with antigen, followed by contacting the DCs with IFN-γ and LPS. The activated DCs can then be suspended in a freezing medium and frozen until ready for use in immunotherapy.

Cryopreserved DCs can be cultured *ex vivo* under conditions effective to generate the percent recovery and percent viability of the cells as compared freshly activated DCs. DCs generated from cryopreserved samples can show similar stability as compared to freshly prepared DCs. Furthermore, comparisons of cryopreserved mature DCs with those of freshly prepared DCs can show virtually identical phenotypes as well as signal secretion profiles. As contemplated herein, DCs can be preserved at both small and large scale for approximately 2 to 24 weeks, in the various freezing media described herein, at temperatures of approximately -70°C to -80°C. At temperatures below about -120°C, the duration of storage can be extended indefinitely or at least beyond 24 weeks without impacting cell recovery, viability, and functionality of the DCs. For example, in certain embodiments, the activated cells can be preserved for at least one year and still retain their ability to produce signal after thawing. The present invention provides for effective recovery and viability profiles upon thawing the cells, and furthermore the cryopreservation conditions described herein do not affect the ability of DCs to retain their signal profiles as explained herein throughout.

In an exemplary embodiment, cryopreservation may be performed after activation of DCs by re-suspending the cells in a freezing medium comprising about 55% plasmalyte, about 40% human serum albumin, and about 5% DMSO. The mixture can then be aliquoted in 1.8 ml vials and frozen at about -80°C in a cryochamber overnight. Vials can then be transferred to liquid nitrogen tanks the following day. After about 2 to 24 weeks of cryopreservation, the frozen DCs can be thawed and examined for their recovery and viability. Recovery of such DCs can be greater than or equal to about 70% with a viability of greater than or equal to about 70%. In other embodiments, DCs or even monocytes can be cryopreserved prior to cell activation.

Alternatively, the procedure for *ex vivo* expansion of hematopoietic stem and progenitor cells is described in U.S. Pat. No. 5,199,942, which is incorporated herein by reference, can be applied to the cells of the present invention. In addition to the cellular growth factors described in U.S. Pat. No. 5,199,942, other factors such as flt3-L, IL-1, IL-3 and c-kit ligand, can be used for culturing and expansion of the cells.

A variety of cell selection techniques are known for identifying and separating cells from a population of cells. For example, monoclonal antibodies (or other specific cell binding proteins) can be used to bind to a marker protein or surface antigen protein found on the cells. Several such markers or cell surface antigens are known in the art.

### Vaccine Formulations

The present invention further includes vaccine formulations suitable for use in immunotherapy. In certain embodiments, vaccine formulations are used for the prevention and/or treatment of a disease, such as cancer and infectious diseases. In one embodiment, the administration to a patient of a vaccine in accordance with the present invention for the prevention and/or treatment of cancer can take place before or after a surgical procedure to remove the cancer, before or after a chemotherapeutic procedure for the treatment of cancer, and before or after radiation therapy for the treatment of cancer and any combination thereof. In other embodiments, the vaccine formulations may be administrated to a patient in conjunction or combination with another composition or pharmaceutical product. It should be appreciated that the present invention can also be used to prevent cancer in individuals without cancer, but who might be at risk of developing cancer.

The administration of a cancer vaccine prepared in accordance with the present invention, is broadly applicable to the prevention or treatment of cancer, determined in part by the selection of antigens forming part of the cancer vaccine. Cancers that can be suitably treated in accordance with the practices of the present invention include, without limitation, cancers of the lung, breast, ovary, cervix, colon, head and neck, pancreas, prostate, stomach, bladder, kidney, bone, liver, esophagus, brain, testicle, uterus and the various leukemias and lymphomas.

In one embodiment, vaccines in accordance with this invention can be derived from the tumor or cancer cells to be treated. For example, in the treatment of lung cancer, the lung cancer cells would be treated as described hereinabove to produce a lung cancer vaccine. Similarly, breast cancer vaccine, colon cancer vaccine, pancreas cancer vaccine, stomach cancer vaccine, bladder cancer vaccine, kidney cancer vaccine and the like, would be produced and employed as immunotherapeutc agents in accordance with the practices for the prevention and/or treatment of the tumor or cancer cell from which the vaccine was produced.

In another embodiment, vaccines in accordance with the present invention could, as stated, also be prepared to treat various infectious diseases which affect mammals, by collecting the relevant antigens shed into a culture medium by the pathogen. As there is heterogenecity in the type of immunogenic and protective antigens expressed by different varieties of organisms causing the same disease, polyvalent vaccines can be prepared by preparing the vaccine from a pool of organisms expressing the different antigens of importance.

In another embodiment of the present invention, the vaccine can be administered by intranodal injection into groin nodes. Alternatively, and depending on the vaccine target, the vaccine can be intradermally or subcutaneously administered to the extremities, arms and legs, of the patients being treated. Although this approach is generally satisfactory for melanoma and other cancers, including the prevention or treatment of infectious diseases, other routes of administration, such as intramuscularly or into the blood stream may also be used.

Additionally, the vaccine can be given together with adjuvants and/or immuno-modulators to boost the activity of the vaccine and the patient's response. Such adjuvants and/or immuno-modulators are understood by those skilled in the art, and are readily described in available published literature.

As contemplated herein, and depending on the type of vaccine being generated, the production of vaccine can, if desired, be scaled up by culturing cells in bioreactors or fermentors or other such vessels or devices suitable for the growing of cells in bulk. In such apparatus, the culture medium would be collected regularly, frequently or continuously to recover therefrom any materials or antigens before such materials or antigens are degraded in the culture medium.

If desired, devices or compositions containing the vaccine or antigens produced and recovered, in accordance with the present invention, and suitable for sustained or intermittent release could be, in effect, implanted in the body or topically applied thereto for a relatively slow or timed release of such materials into the body.

Other steps in vaccine preparation can be individualized to satisfy the requirements of particular vaccines. Such additional steps will be understood by those skilled in the art. For example, certain collected antigenic materials may be concentrated and in some cases treated with detergent and ultracentrifuged to remove transplantation alloantigens.

### Combination Therapy

The present invention provides an effective therapy to treat cancer wherein the therapy includes changing the immune response in the tumor so that the immune cells in the tumor site are more effective in attacking the tumor cells. In some instances, the effective therapy includes improving the migration and activity of immune cells in the tumor site. In one embodiment, the invention provides compositions and methods of using a dendritic cell vaccine in combination with an inhibitor of one or more of HER2 and HER3 as a treatment regimen to treat cancer. In another embodiment, the treatment regimen comprises the use of a dendritic cell vaccine, an inhibitor of one or more of HER2 and HER3, and a chemokine modulator. In one embodiment, the chemokine modulator is a chemokine-activating agent. An example of a chemokine-activating agent is a TLR8 agonist.

In one embodiment, the invention provides compositions and methods of using a dendritic cell vaccine in combination with blockage of HER-2 and HER-3 as a treatment regimen to treat cancer. In another embodiment, the invention provides compositions and methods of using a dendritic cell vaccine in combination with blockage of HER-2 and HER-3 with TNF-α and IFN-γ. In another embodiment, the invention provides compositions and methods of blocking both of HER-2 and HER-3 with the addition of TNF-α and IFN-γ as a treatment regimen to treat cancer.

In one embodiment, the treatment regimen of the invention can be used to treat cancer and therefore can be considered as a type of anti-cancer therapy. In another embodiment, the treatment regimen of the invention can be used in the context of a combination therapy with another anti-cancer or anti-tumor therapy including but not limited to surgery, chemotherapy, radiation therapy (e.g. X ray), gene therapy, immunotherapy, hormone therapy, viral therapy, DNA therapy, RNA therapy, protein therapy, cellular therapy, and nanotherapy.

In one embodiment, the invention involves the treatment regimen of the invention in combination with another cancer medicament for the treatment or prevention of cancer in subjects. The other cancer medicament is administered in synergistic amounts or in various dosages or at various time schedules with the treatment regimen of the invention. The invention also relates to kits and compositions concerning the combination of treatment regimens of the invention alone or in combination with a desired cancer medicament.

In one embodiment, the treatment regimen of the invention is used prior to receiving another anti-cancer therapy. In another embodiment, the treatment regimen of the invention is used concurrently with receiving another anti-cancer therapy. In another embodiment, the treatment regimen of the invention is used after receiving another anti-cancer therapy.

In some embodiments, the present invention provides a method of treating breast cancer that is negative for ER in a subject. In some embodiments, the present invention provides a method of treating breast cancer that is negative for ER and positive for HER2 in a subject. In some embodiments, the breast cancer is a metastatic breast cancer. In some embodiments, the breast cancer is at stage I, stage II, or stage III.

In another embodiment, the treatment regimen of the invention may be used in combination with existing therapeutic agents used to treat cancer. In order to evaluate potential therapeutic efficacy of the treatment regimen of the invention in combination with the antitumor therapeutics described elsewhere herein, these combinations may be tested for antitumor activity according to methods known in the art.

In one aspect, the present invention contemplates that the treatment regimen of the invention may be used in combination with a therapeutic agent such as an anti-tumor agent including but is not limited to a chemotherapeutic agent, an anti-cell proliferation agent or any combination thereof.

The invention should not limited to any particular chemotherapeutic agent. Rather, any chemotherapeutic agent can be used with the treatment regimen of the invention. For example, any conventional chemotherapeutic agents of the following non-limiting exemplary classes are included in the invention: alkylating agents; nitrosoureas; antimetabolites; antitumor antibiotics; plant alkyloids; taxanes; hormonal agents; and miscellaneous agents.

Alkylating agents are so named because of their ability to add alkyl groups to many electronegative groups under conditions present in cells, thereby interfering with DNA replication to prevent cancer cells from reproducing. Most alkylating agents are cell cycle non-specific. In specific aspects, they stop tumor growth by cross-linking guanine bases in DNA double-helix strands. Non-limiting examples include busulfan, carboplatin, chlorambucil, cisplatin, cyclophosphamide, dacarbazine, ifosfamide, mechlorethamine hydrochloride, melphalan, procarbazine, thiotepa, and uracil mustard.

Anti-metabolites prevent incorporation of bases into DNA during the synthesis (S) phase of the cell cycle, prohibiting normal development and division. Non-limiting examples of antimetabolites include drugs such as 5-fluorouracil, 6-mercaptopurine, capecitabine, cytosine arabinoside, floxuridine, fludarabine, gemcitabine, methotrexate, and thioguanine.

There are a variety of antitumor antibiotics that generally prevent cell division by interfering with enzymes needed for cell division or by altering the membranes that surround cells. Included in this class are the anthracyclines, such as doxorubicin, which act to prevent cell division by disrupting the structure of the DNA and terminate its function. These agents are cell cycle non-specific. Non-limiting examples of antitumor antibiotics include dactinomycin, daunorubicin, doxorubicin, idarubicin, mitomycin-C, and mitoxantrone.

Plant alkaloids inhibit or stop mitosis or inhibit enzymes that prevent cells from making proteins needed for cell growth. Frequently used plant alkaloids include vinblastine, vincristine, vindesine, and vinorelbine. However, the invention should not be construed as being limited solely to these plant alkaloids.

The taxanes affect cell structures called microtubules that are important in cellular functions. In normal cell growth, microtubules are formed when a cell starts dividing, but once the cell stops dividing, the microtubules are disassembled or destroyed. Taxanes prohibit the microtubules from breaking down such that the cancer cells become so clogged with microtubules that they cannot grow and divide. Non-limiting exemplary taxanes include paclitaxel and docetaxel.

Hormonal agents and hormone-like drugs are utilized for certain types of cancer, including, for example, leukemia, lymphoma, and multiple myeloma. They are often employed with other types of chemotherapy drugs to enhance their effectiveness. Sex hormones are used to alter the action or production of female or male hormones and are used to slow the growth of breast, prostate, and endometrial cancers. Inhibiting the production (aromatase inhibitors) or action (tamoxifen) of these hormones can often be used as an adjunct to therapy. Some other tumors are also hormone dependent. Tamoxifen is a non-limiting example of a hormonal agent that interferes with the activity of estrogen, which promotes the growth of breast cancer cells.

Miscellaneous agents include chemotherapeutics such as bleomycin, hydroxyurea, L-asparaginase, and procarbazine that are also useful in the invention.

An anti-cell proliferation agent can further be defined as an apoptosis-inducing agent or a cytotoxic agent. The apoptosis-inducing agent may be a granzyme, a Bcl-2 family member, cytochrome C, a caspase, or a combination thereof. Exemplary granzymes include granzyme A, granzyme B, granzyme C, granzyme D, granzyme E, granzyme F, granzyme G, granzyme H, granzyme I, granzyme J, granzyme K, granzyme L, granzyme M, granzyme N, or a combination thereof. In other specific aspects, the Bcl-2 family member is, for example, Bax, Bak, Bcl-Xs, Bad, Bid, Bik, Hrk, Bok, or a combination thereof.

In additional aspects, the caspase is caspase-1, caspase-2, caspase-3, caspase-4, caspase-5, caspase-6, caspase-7, caspase-8, caspase-9, caspase-10, caspase-11, caspase-12, caspase-13, caspase-14, or a combination thereof. In specific aspects, the cytotoxic agent is TNF-α, gelonin, Prodigiosin, a ribosome-inhibiting protein (RIP), Pseudomonas exotoxin, Clostridium difficile Toxin B, Helicobacter pylori VacA, Yersinia enterocolitica YopT, Violacein, diethylenetriaminepentaacetic acid, irofulven, Diptheria Toxin, mitogillin, ricin, botulinum toxin, cholera toxin, saporin 6, or a combination thereof.

In one embodiment, the treatment regimen of the invention is used in combination with an anti-tumor agent wherein the anti-tumor agent is an antitumor alkylating agent, antitumor antimetabolite, antitumor antibiotics, plant-derived antitumor agent, antitumor platinum complex, antitumor campthotecin derivative, antitumor tyrosine kinase inhibitor, monoclonal antibody, interferon, biological response modifier, hormonal anti-tumor agent, anti-tumor viral agent, angiogenesis inhibitor, differentiating agent, PI3K/mTOR/AKT inhibitor, cell cycle inhibitor, apoptosis inhibitor, hsp 90 inhibitor, tubulin inhibitor, DNA repair inhibitor, anti-angiogenic agent, receptor tyrosine kinase inhibitor, topoisomerase inhibitor, taxane, agent targeting Her-2, hormone antagonist, agent targeting a growth factor receptor, or a pharmaceutically acceptable salt thereof. In some embodiments, the anti-tumor agent is citabine, capecitabine, valopicitabine or gemcitabine. In some embodiments, the anti-tumor agent is selected from the group consisting of Avastin, Sutent, Nexavar, Recentin, ABT-869, Axitinib, Irinotecan, topotecan, paclitaxel, docetaxel, lapatinib, Herceptin, lapatinib, tamoxifen, a steroidal aromatase inhibitor, a non-steroidal aromatase inhibitor, Fulvestrant, an inhibitor of epidermal growth factor receptor (EGFR), Cetuximab, Panitumimab, an inhibitor of insulin-like growth factor 1 receptor (IGF1R), and CP-751871.

In one embodiment, the anti-tumor agent is a chemotherapeutic agent. A chemotherapeutic agent as used herein is a chemical compound useful in the treatment of cancer. Examples of chemotherapeutic agents include alkylating agents such as thiotepa and cyclosphosphamide (CYTOXAN); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL); beta-lapachone; lapachol; colchicines; betulinic acid; a camptothecin (including the synthetic analogue topotecan (HYCAMTIN), CPT-11 (irinotecan, CAMPTOSAR), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e. g., calicheamicin, especially calicheamicin gamma1I and calicheamicin omegaI1 (see, e.g., Agnew, Chem Intl. Ed. Engl., 33:183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, doxorubicin (including ADRIAMYCIN, morpholino-doxorubicin, cyanomorpholino-doxorubicin, 2-pyrrolino-doxorubicin, doxorubicin HCl liposome injection (DOXIL), liposomal doxorubicin TLC D-99 (MYOCET), peglylated liposomal doxorubicin (CAELYX), and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate, gemcitabine (GEMZAR), tegafur (UFTORAL), capecitabine (XELODA), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK polysaccharide complex (JHS Natural Products, Eugene, Oreg.); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; dacarbazine; mannomustine; mitobronitol; mitolactol; pipobroman; gacytosine; arabinoside ("Ara-C"); thiotepa; taxoid, e.g., paclitaxel (TAXOL), albumin-engineered nanoparticle formulation of paclitaxel (ABRAXANE), and docetaxel (TAXOTERE); chloranbucil; 6-thioguanine; mercaptopurine; methotrexate; platinum agents such as cisplatin, oxaliplatin, and carboplatin; vincas, which prevent tubulin polymerization from forming microtubules, including vinblastine (VELBAN), vincristine (ONCOVIN), vindesine (ELDISINE, FILDESIN), and vinorelbine (NAVELBINE); etoposide (VP-16); ifosfamide; mitoxantrone; leucovovin; novantrone; edatrexate; daunomycin; aminopterin; ibandronate; topoisomerase inhibitor RFS 2000; difluorometlhylornithine (DMFO); retinoids such as retinoic acid, including bexarotene (TARGRETIN); bisphosphonates such as clodronate (for example, BONEFOSO or OSTAC), etidronate (DIDROCAL), NE-58095, zoledronic acid/zoledronate (ZOMETA), alendronate (FOSAMAX), pamidronate (AREDIA), tiludronate (SKELID), or risedronate (ACTONEL); troxacitabine (a 1,3-dioxolane nucleoside cytosine analog); antisense oligonucleotides, particularly those that inhibit expression of genes in signaling pathways implicated in aberrant cell proliferation, such as, for example, PKC-alpha, Raf, H-Ras, and epidermal growth factor receptor (EGF-R); vaccines such as THERATOPE.RTM. vaccine and gene therapy vaccines, for example, ALLOVECTIN vaccine, LEUVECTIN vaccine, and VAXID vaccine; topoisomerase 1 inhibitor (e.g., LURTOTECAN); rmRH (e.g., ABARELIX); BAY439006 (sorafenib; Bayer); SU-11248 (Pfizer); perifosine, COX-2 inhibitor (e.g., celecoxib or etoricoxib), proteosome inhibitor (e.g., PS341); bortezomib (VELCADE); CCI-779; tipifarnib (R11577); orafenib, ABT510; Bcl-2 inhibitor such as oblimersen sodium (GENASENSE); pixantrone; EGFR inhibitors; tyrosine kinase inhibitors; and pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN) combined with 5-FU and leucovovin.

These methods described herein are by no means all-inclusive, and further methods to suit the specific application will be apparent to the ordinary skilled artisan. Moreover, the effective amount of the compositions can be further approximated through analogy to compounds known to exert the desired effect.

### EXPERIMENTAL EXAMPLES

The invention is further described in detail by reference to the following experimental examples. These examples are provided for purposes of illustration only, and are not intended to be limiting unless otherwise specified. Thus, the invention should in no way be construed as being limited to the following examples, but rather, should be construed to encompass any and all variations which become evident as a result of the teaching provided herein.

Without further description, it is believed that one of ordinary skill in the art can, using the preceding description and the following illustrative examples, make and utilize the present invention and practice the claimed methods. The following working examples therefore, specifically point out the preferred embodiments of the present invention, and are not to be construed as limiting in any way the remainder of the disclosure.

### Example 1: A cryopreserved, pre-activated, multi-dose dendritic cell vaccine

To be able to produce these large scale vaccines, a process was developed to produce fully activated DC1 vaccines pulsed with tumor antigen whereby the fully activated DC1 vaccines are cryopreserved as multi-dose syringe ready 6 pack DC1 vaccines. The DC1 are cryopreserved activated as DC1 as described elsewhere herein. For example, they are cryopreserved in 55% Plasmalyte medium with 40% Human serum albumin and 5% DMSO. These vaccines have been generated and extensively tested in the laboratory and consistently meet quality standards set by the FDA for administration to patients.

The materials and methods employed in the experiments and examples disclosed herein are now described.

### Preparation of fully activated DC for cryopreservation

Freshly elutriated myeloid monocytes were cultured in 6 well microplates (12x10⁶ cells/well). Culture medium consisted of Serum Free Medium (SFM Invitrogen Carlsbad CA). The final concentration of added GMCSF was 50ng/ml and of IL4 is 1000 U/ml. Cells were cultured overnight at 37° C in 5% CO₂. In some batches, the cells were pulsed with the adequate peptides after 16-20 hr and cultured for additional 6-8 hr, after which 1000U/ml IFN-γ was added. Dendritic cells were matured with TLR agonist LPS (TLR 4, 10ng/ml) or R848 (TLR8, 1µg/ml). The maturation time was at least about 6hr. After that, the TLR agonist-activated DCs were ready for cryopreservation or immediate use.

To induce the production of the Thl-polarizing cytokine IL-12, the DCs are activated with combinations of the cytokine IFN-γ, or the TLR agonists bacterial LPS and/or R848. This should induced T cells that produce IFN-γ. Alternatively, the DCs can be activated with combinations of ATP, bacterial LTA, LPS and prostaglandin E2 (PGE2). This can cause IL-23, IL-6 and IL-1β to be amplified, leading an immune response dominated by IL-17 and IL-22-secreting Th17 cells.

### Cryopreservation of DC

DCs were harvested by gentle scraping. All medium and the cells were kept at wet ice at all times. Cells were gently washed by centrifugation at about 800RPM for 10 min. Cells (e.g., 10 x10⁶ cells) were cryopreserved in freezing medium of plasmalyte 55%, human serum albumin 40% with 5% DMSO and stored in liquid nitrogen.

The results of the experiments presented herein are now described.

### multi-dose DC1 vaccines

Experiments were performed to assess the recovery, viability and sterility of the cells. Briefly, peripheral blood monocytes were obtained by combined leukapheresis the counter current elutriation and were cultured in serum free medium with GM-CSF and IL-4 overnight. The next day they were pulsed with HER-2 peptides then IFN-γ followed by LPS. The DC1 were harvested at 40 hours and cryopreserved in freezing medium of plasmalyte 55%, human serum albumin 40% with 5% DMSO and stored in liquid nitrogen. Following 1 week they were thawed and release criteria were obtained including viability, yield, endotoxin testing, and sterility cultures. All 12 cultures had no bacterial growth and <0.1 EU endotoxin. Figure 1 demonstrates the viability and yield of cryopreserved DC1. As can be seen in Figure 1, recovery of cells was on average 89% and viability was 95% when cells were directly thawed and counted. These data demonstrate the cryopreservation and viability of the DC1 vaccine is maintained in medium containing below the FDA allowable 7.5% DMSO.

It was observed that cell function was maintained following crypreservation. IL-12 and Th1 chemokines were produced for 36 hours post thaw from multi-dose DC1 vaccines. Thawed cells produced high levels of IL-12 from about 6 hours post thaw through 36 hours. These levels of IL-12 are comparable to prepared DC1 vaccines made from cryopreserved monocytes.

The results presented herein demonstrate that cryopreserved, preactivated, multi-dose dendritic cell vaccine sensitized against the HER-2 tumor target antigen on breast cancer is therapeutic. However, the invention is applicable to a variety of additional pathophysiological conditions.

The multi-dose syringe ready pack DC1 vaccines can be used in HER-2 non-expressing breast cancer. HER-2 is expressed on approximately 25% of all breast cancers. Breast cancers that do not produce detectable levels of HER-2 may not be susceptible to vaccination. To address this limitation, additional target proteins can be added to the vaccine. For example, many breast cancers that do not produce high levels of HER-2 instead produce other, related proteins including HER-1 and HER-3. Without wishing to be bound by any particular theory, it is believed that adding these other proteins to the vaccine would allow the targeting of these other breast cancer phenotypes.

The multi-dose syringe ready pack DC1 vaccines can be used in other cancer types besides breast cancer. Anticipated target proteins such as HER-1, HER-2 and HER-3 can also be present on other types of cancer including ovarian, prostate, pancreatic, colorectal, gastric, head and neck and non-small cell lung carcinoma, as well as other common cancers.

The multi-dose syringe ready pack DC1 vaccines can be used to treat chronic infectious diseases, including but not limited to, chronic infections like HIV or hepatitis virus C. Here, proteins specific for these viruses would replace the HER-2 or other cancer proteins to mobilize the patient's immune response against these persistent infections. It is possible that the enhanced immunity would greatly reduce viral load and attendant disease symptoms and progression, or could possibly help clear the infection entirely.

The multi-dose syringe ready pack DC1 vaccines can be used to treat autoimmune diseases. Diseases like rheumatoid arthritis and Lupus occur when the immune system mistakenly attacks the body's own normal tissues. Whereas the current vaccine/immunotherapy formulation is designed to initiate and strengthen immune responses, without wishing to be bound by any particular theory, it is believed that in vitro signals can be provided to the DCs during vaccine production that induce these cells to switch off pathological immune responses.

### Example 2: Cryopreservation of Activated DC1 Makes Large Scale Dendritic Cell Vaccines Feasible in Cancer Therapy

Dendritic cell-based vaccine therapy is a promising directed therapy against a variety of cancers. While a variety of strategies have been employed to mature DCs to a phenotype that optimizes sensitization of CD4+ and CD8+ T cells to recognize tumor epitopes and elicit antitumor immunity, experiments were designed to utilize a method that employs the rapid maturation of monocytes in serum free media (SFM) using Interferon gamma (IFN-γ) and lipopolysaccharide (LPS), a toll-like receptor (TLR) 4 agonist, resulting in mature DCs capable of polarizing the immune response to a Th1-type response and eliciting sensitization via an IL-12 dependent mechanism. Results demonstrate the potential for this vaccine strategy to be used as an adjunct therapy in early breast cancer. Cryopreservation of dendritic cells (DCs) in a matured state permits easier production of and accessibility to personalized therapy.

### Rapid maturation of dendritic cells

Both freshly matured DCs (DCIs) and DCs cryopreserved in a mature state (cryoDCs) and subsequently thawed were compared for viability and recover as well as for phenotypic maturity as determined by expression of cell surface markers by flow cytometry. Function of dendritic cells was determined by measuring production of various cytokines, in particular, interleukin 12p70 (IL-12p70). The ability of these cells to stimulate naive CD4+ and CD8+ tells was also assessed.

There was no significant difference in the viability (p=.4807), and recovery (p=.1220) (Figure 2).

Both populations had similar initial (7 hours post LPS addition) IL-12 p70 secretion (p=.0768). The populations continued to exhibit comparable secretion levels of IL-12 p70 over a 30 hour observation period with no significant differences between the populations. (Figure 3).

There was no significant difference between populations and production of IL-1beta (p=0.7690), IL-1 alpha (p=0.0841), Rantes (p=0.902), MDC (p=0.1514), IL-10 (p=.1937), MIP-1alpha (p=.2673), IP-10 (p=0.7366), IL-6 (p=0.24), IL-5 (p=0.0735), TNF-beta (p=0.9422), IL-15(p=0.8878), MIP-1beta (p=0.9217), TNF-alpha (p=0.8972), IL-8(p=0.7844). (Figure 4).

Cell surface markers denoting DC maturity between DC1s and cryoDCs exhibit no significant difference in expression. Both populations elicited nonspecific alloantigen CD4+ T cell responses as well as antigen-specific CD8+ T cells recognition with a Th1 polarized response. CD80, 83, and 86 demonstrate DC maturation, expression revealed no significant difference. Functional abilities were induced by CD4+ T cells from different donors co-cultured with each population resulting in a nonspecific alloantigen response and CD4+ T cells secreting INF-gamma (DC1s 107.40 ng/mL and cryoDCls 129.23 ng/mL). Antigen specific sensitizations initiated by co-culturing the two populations with tumor antigen specific CD8+ T cells elicit comparable IFN-γ secretions for both groups and result in a TH1 polarized response. (Figure 5).

The results presented herein demonstrate that rapid maturation method of DC1 can be cryopreserved functionally matured and maintains, phenotype and function, thus can be used to manufacture syringe ready DC1 for use world-wide in cancer therapy.

In conjunction with maintaining the DC1 phenotype to drive a Th1 polarized immune response, the results presented herein demonstrate that the cryoDCs maintained the ability to primarily sensitize T cells. This also likely relates to the maturation strategy, as the DC1s matured with IFN-gamma and LPS exhibit an enhanced ability to primarily sensitize CD 4+ T cells compared to cytokine matured DCs.

The present protocol for cryopreserved mature DC preparations can be easily adapted to current good manufacturing practice guidelines thereby increasing the availability of emerging therapy.

### Example 3: Cytokines from CD4 T cells and Herceptin make high HER-2-expressing breast cells susceptible to killing by CD8 T cells

It has been demonstrated that that high HER-2-expressing breast cancer cells down-regulate molecules on the cell surface that make these cancer cells visible to CD8 T cells and allow them to be killed by these immune cells. It has been shown that the cytokines interferon-gamma (IFN-γ) and tumor necrosis factor-alpha (TNF-α) produced by CD4 cells, when combined with Herceptin, cause the intermediate and high HER-2-expressing breast cancer cells to increase their Class I molecule expression. As a result of this, the CD8 T cells are able to better see the breast cancer cells and kill them or to produce cytokines to kill them.

Trials were designed to assess the therapeutic effects of using Herceptin in combination with dendritic cell vaccines (e.g., DC1 vaccines).

A phase I DCIS vaccine trial combining Herceptin with DC1 vaccines was designed. For example, a Phase I trial was designed for patients with high HER-2-expressing DCIS to receive a DC1 vaccine combined with 2 doses of Herceptin at week 1 and week 4. Without wishing to be bound by any particular theory, it is believed that this combination will increase the complete response rate from 30% to greater than 50% in patients with HER-2-expressing DCIS.

In addition, a Phase III DCIS vaccine trial was designed. For example, a vaccine trial was developed to prevent recurrence of breast cancer in patients with estrogen independent (ER^{-negative}), HER-2^{-positive} DCIS. There are three treatment arms in the study: 1) a standard therapy (surgery and radiation), 2) receiving a DC1 vaccine before surgery, and 3) receiving a DC1 vaccine plus Herceptin before surgery. Without wishing to be bound by any particular theory, those patients who have complete responses to the treatment can avoid radiation after surgery. It is also believed that this trial serves demonstrate the prevention of recurrence in patients with DCIS using a vaccine.

In addition, a phase I neoadjuvant DC1 vaccine in combination with Herceptin in patients with early invasive HER-2^{-positive} breast cancer was designed. For example, a Phase I trial was designed to test whether the combination of Herceptin and vaccines along with a chemokine modulator (e.g., a chemokine-activating agent) can eliminate small HER-2-expressing invasive breast cancers prior to surgery and avoid the need for chemotherapy. Without wishing to be bound by any particular theory, it is believed that this neoadjuvant (before surgery) strategy, with the possibility of adding an immune antibody that takes the brakes off the immune response, may eliminate the need for toxic chemotherapies for the treatment of breast cancer and therefore make immune therapy the standard of care for this disease. That is, the treatment regimen disclosed herein provides a step forward in the quest to eradicate breast cancer using the natural immune response, which can be restored with vaccines regimens of the invention. The regiments discussed herein can drive immune cells into the tumor by changing the immune response in the tumor and enable the immune cells to work longer by taking the brakes off the cells. It is believed that combining DC1 vaccines with Herceptin and also adding the chemokine modulator improves the migration and activity of the immune cells within the tumor in the breast.

Without wishing to be bound by any particular theory, it is believed that in many cancers, if there are large numbers of good immune-fighting cells present, those patients do better with any type of therapy. This means that if one gets immune cells into the tumor to fight it before it is removed, the patient likely does better and responds better to other therapies, including surgery, chemotherapy, and radiation.

Without wishing to be bound by any particular, an effective therapy to treat cancer would include agents that rapidly change the immune response in the tumor prior to surgery to improve outcomes for patients with breast cancer and other types of cancer. This strategy can be applied to a variety of cancers including but is not limited to colon cancer, melanoma, lung, brain, pancreas, prostate, esophagus, and the like.

Experiments were designed to develop immune fluorescence assays to measure and quantify the types of immune cells that migrate into the breast after vaccination, the types of chemokines that are made to bring cells in, and the molecules they express to help eliminate cancer cells. These assays allow multiple cell types to be visualized at the same time and show where they are located in the tumor microenvironment. It has been observed that at least in some of the vaccinated patients, their tumors produced chemokines to recruit cells into the environment to kill the tumor cells.

### Example 4: Combination of blockade of HER-2 and HER-3

The results presented herein demonstrate that the combination of blocking HER-2 and HER-3 is extremely effective with anti-HER-2 CD4 Th1cells in causing permanent tumor senescence in HER-2 expressing breast cancers.

It was observed that the combination of blockade of HER-2 and HER-3 together and the addition of TNF-α and IFN-γ from CD4 Th1 cells causes almost complete senescence of HER-2 expressing breast cancer. It has been verified in several different cells lines both high and intermediate expressing HER-2+ breast cancer cells. The results demonstrate that this combination can be potent for prevention and prevention of recurrence.

The materials and methods employed in these experiments are now described.

### Cell culture and treatments

Human breast cancer cell lines SK-BR-3, BT-474, MCF-7, T-47D, HCC-1419 and MDA-MB-231 were obtained from the American Type Culture Collection (Manassas, VA) and grown in RPMI-1640 (Life technologies, Grand Island, NY) supplemented with 10% FBS (Cellgro, Herndon, VA). JIMT-1 cells were obtained from Ohio State University (Columbus, OH) and were grown in the same complete medium. Normal immortalized MCF-10 cells were obtained from the Karmanos Cancer Institute (Detroit, MI) and grown in RPMI-1640 supplemented with 10 mM HEPES, 10 µg/ml insulin, 20 ng/ml EGF, 100 ng/ml cholera toxin, 30 mM sodium bicarbonate, 0.5 µg/ml hydrocortisone, and 5% fetal horse serum. All cells were grown at 37°C in a humidified 5% CO₂ incubator.

Three hundred thousand of breast cancer cells were treated for 5 days with the indicated concentrations of human recombinant TNF-γ (R&D Systems, Minneapolis, MN) and human recombinant IFN-γ(R&D Systems) and then cultured for 2 more passages in absence of cytokines. Cells were subjected to senescence associated β-galactosidase enzyme (SA-β-gal) detection or lysed and subjected to western blot analysis for p15INK4b and p16INK4a.

In the some cases, the cells were treated with 10 ug/ml trastuzumab and pertuzumab (Genentech, San Francisco, CA) for the indicated times. This treatment was combined with cytokines or with human recombinant heregulin (R&D Systems).

The same amount of cells was also cultured in the lower chamber of a transwell system (BD Biosciences, San Jose, CA) with co-culture of 10x10⁵ human CD4⁺ T-cells and 10⁵ mature (i.e. type 1 polarized) or immature human DCs in the upper chamber. DCs and CD4⁺ T-cells were obtained from select trial subjects (Sharma et al., 2012 Cancer 118: 4354-4362). Mature and immature DCs were pulsed with Class II-derived HER2 or control irrelevant (BRAF and survivine) peptides (20 ng/ml) for 5 days at 37°C. Control wells contained CD4⁺ T-cells only. In addition, 0.5x10⁵ cells were incubated in the presence of DC/CD4⁺ T-cell co-culture supernatants for 5 days at 37°C. In both approaches, cells were then cultured for 2 more passages in absence of cytokines and subjected to senescence studies (SA-β-gal activity at pH 6 and p15INK4b and p16INK4a western blot) or apoptosis studies (cleaved caspase-3 western blot). The following antibodies were added to cells 60 min before incubation with the co-culture of DC and CD4⁺ T-cells to neutralize Th1-elaborated cytokines: polyclonal goat IgG antihuman TNF-γ (0.06 µg/ml per 0.75 ng/ml TNF-α) and IFN-γ (0.3 µg/ml per 5 ng/ml IFN-γ), and goat IgG isotype as the corresponding negative control (all from R&D Systems).

### Plasmid transfections

MDA-MB-231 cells were transiently transfected for 48 h with 2 µg of the wt HER2 expression vector (pcDNAHER2). As control, cells were transfected with 2 µg of the empty vector (pcDNA3). Both vectors were kindly provided by Dr. Mark Greene (University of Pennsylvania, Philadelphia, PA). The cells were transfected in complete medium without antibiotics with Turbofect (Thermo Scientific, Waltham, MA). Transfection efficiency was evaluated by western blot 48 h after transfection.

### RNA interference (RNAi) transfections

Small interfering RNA (siRNA) SMART Pool: ON TARGET Plus HER2 siRNA, HER3 siRNA and SMART Pool: ON-TARGETplus Non-targeting Pool were purchased from Dharmacon-Thermo Scientific. The following target sequences were used: HER2: UGGAAGAGAUCACAGGUUA (SEQ ID NO.1), GAGACCCGCUGAACAAUAC (SEQ ID NO.2), GGAGGAAUGCCGAGUACUG (SEQ ID NO.3), GCUCAUCGCUCACAACCAA (SEQ ID NO.4); HER3: GCGAUGCUGAGAACCAAUA (SEQ ID NO.5), AGAUUGUGCUCACGGGACA (SEQ ID NO.6), GCAGUGGAUUCGAGAAGUG (SEQ ID NO.7), UCGUCAUGUUGAACUAUA (SEQ ID NO.8); Non-targeting: UGGUUUACAUGUCGACUAA, UGGUUUACAUGUUGUGUGA, UGGUUUACAUGUUUUCUGA (SEQ ID NO.9), UGGUUUACAUGUUUUCCUA (SEQ ID NO.10). Three hundred thousand cells were transfected with siRNA sequences (25 nM) using RNAi Max Lipofectamine (Life Technologies) in serum free medium, and after 1 h the medium was supplemented with 10% FBS. Sixteen hours later, cells were subjected to 48 h of serum starvation followed by various designated treatments and western blot to check expression levels.

### SA-β-gal activity at pH 6

Cells were washed twice in PBS, fixed in 3% formaldehyde, and washed again in PBS. The cells were incubated overnight at 37°C (without CO₂) with freshly prepared senescence associated acidic β-galactosidase (SA-β-gal) staining solution from Millipore (Billerica, MA) as manufacturer's instructions.

### Western blot analysis

Lysates were prepared from MCF-10A, SK-BR-3, and MCF-7, T-47 D or MDA-MB-231 cells. Cells were lysed in a buffer containing 50 mM Tris (pH 7.4), 150 mM NaCl, 1 mM EDTA, 1 mM EGTA, 10% glycerol, 70% Tergitol, 0.1% SDS, 1 mM Mg₂Cl and protease inhibitor cocktail Sigma-Aldrich (St. Louis, MO). Lysates were centrifuged at 12,000xg for 15 min at 4°C. Proteins were solubilized in sample buffer (Life Technologies) and subjected to SDS-PAGE. Proteins were electroblotted onto PVDF. Membranes were immunoblotted with the following antibodies: p15INK4b (K-18), p16INK4a (50.1), IFN-γRα (C-20), HER3 (C-17) all from Santa Cruz Biotechnology (Santa Cruz, CA); Vinculin (V9131) from Sigma-Aldrich; HER2 (29D8), Cleaved Caspase-3 (8G10) and TNF-R1 (C25C1) from Cell Signaling Technologies (Danvers, MA). After washing, membranes were incubated with HRP-conjugated secondary antibody (Bio-Rad, Hercules, CA). Bands were visualized by using the enhanced chemiluminescence (ECL) Western blotting detection system Western Blot Analysis.

### Flow cytometric analysis of cell death

SK-BR-3 cells were untreated, treated with IFN-γ (100 U/ml) and TNF-α (10ng/ml), treated with trastuzumab (10µg/ml) and pertuzumab (10µg/ml), or treated with a combination of IFN-γ, TNF-α, trastuzumab, and pertuzumab, for 24 hours. After incubation, apoptosis induction was determined using FITC-Annexin V apoptosis detection kit (BD biosciences) according to manufacturer's instructions. Briefly, untreated and treated SKBR-3 cells were collected, washed with PBS and resuspended in Annexin V binding buffer at a concentration of 1x10⁶ cells/ml. 100µl of cell suspension was incubated with 5µl PI and 5µl FITC-annexin V for 15 min at room temperature in the dark. After incubation, 150µl annexin V binding buffer was added and apoptosis induction was analyzed using a BD Accuri C6 flow cytometry (BD Biosciences) and data was analyzed with CFlow Plus software. UV-irradiated cells were used as positive controls.

### Tumorigenesis studies

For xenograft experiments, SK-BR-3 (2 × 10⁶ cells/mouse in 200 µl PBS) were injected into the flanks of six-week old female oathymic (nude) mice (*Foxnnu,* Harlam Laboratories, 5 mice/group). When the tumors were palpable, the animals were treated *s.c.* with trastuzumab and pertuzumab (30 ug/kg) and then injected *s.c.* twice a week with hrTNF-α and hrIFN-γ (10 ng/kg). Tumor formation was monitored by palpation and tumor volume in mm³ was determined with a caliper twice a week: width2 x length/2. All animal experiments were carried out in compliance with the institutions guidelines.

The results of the experiments are now described.

### Th1 cytokines TNF-α and IFN-γ synergize to Induce senescence in breast cancer cells

SK-BR-3 cells were incubated with human recombinant tumor necrosis factor alpha (TNF-α) and interferon gamma (IFN-γ) alone or combined for 5 days at 37°C to study if the elaborated cytokines produced by the immune system cells could induce a specific senescence response in tumor cells. The cells were then cultured for 2 more passages and subjected to senescence studies. The combination of both cytokines resulted in senescence induction of SK-BR-3 cells, evidenced by increased senescence associated acidic β-galactosidase (SA-β-gal) staining (Figure 6A) and higher expression of the senescence-associated markers p15INK4b and p16INK4a (Figure 6B) compared to the control untreated cells or each cytokine alone detected by western blot. Similar results were obtained in BT-474, MCF-7 and T-47D breast cancer cell lines (data not shown). Thus, T-47D cells were treated with several concentrations of TNF-α, from 10 to 100 ng/ml, and IFN-γ, from 100 to 1000 U/ml, or in combination. It was observed that the induction of the senescence phenotype was dose dependent (Figure 6C). Similar results were found in SK-BR-3, BT-474 and MCF-7 cells.

### Inverse correlation between the HER2 expression level in breast cancer cells and the Th1 cytokines TNF-α and IFN-γ doses required to induce senescence

Experiments were designed to determine whether the HER2 expression level plays a role in the senescence induced by the treatment with TNF-α and IFN-γ for 5 days at 37°C followed by 2 more passages without the cytokines in SK-BR-3, BT-474, MCF-7, T-47D and MDA-MB-231 breast cancer cells. In fact, the amount of SA-β-gal positive cells was augmented in the high HER2 expressing cell lines (SK-BR-3, Figure 6D and BT-474, data not shown), with lower doses of TNF-α and IFN-γ compared to the intermediate HER2 expressing cell lines (T-47D, Figure 6D and MCF-7, data not shown) with higher cytokines concentrations. However even highest concentration of TNF-α and IFN-γ could not induce senescence in the low HER2-expressing cell line MDA-MB-231 (Figure 6D). These results clearly evidence a correlation between the TNF-α and IFN-γ inducing senescence and the HER2 expression level.

### HER2 is required for Th1 cytokines TNF-α and IFN-γ mediated senescence and apoptosis in MDA-MB-231 breast cancer cells

Only when MDA-MB-231 cells were stably transfected with a wild type HER2 plasmid (pcDNAHER2) and treated with high concentrations of TNF-α (200 ng/ml) and IFN-γ (2000 U/ml) for 5 days at 37°C followed by 2 more passages in absence of cytokines, there was a strong increase in SA-β-gal positive cells (Figure 7A) and in p15INK4b expression (Figure 7B). Notably, as it was discovered that not only there was relatively higher blue senescent cells when HER2-MDA-MB-231 cells were cultured with highest concentrations of TNF-α and IFN-γ, but also there was significantly less amount of cells, the experiment was repeated and the cells were subjected to western blot to detect active caspase-3 (Figure 7B). The treatment of cells transfected with the control empty vector (pcDNA3) with increasing concentrations of TNF-α, from 75 to 200 ng/ml, and IFN-γ, from 750 to 2000 U/ml, in combination in the same conditions described above, had no effect on senescence induction assessed by SA-β-gal staining (Figure 7A) or p15INK4b and cleaved caspase 3 expressions (Figure 7B). This finding reinforces that HER2 is required in the mechanism of TNF-α and IFN-γ inducing senescence and apoptosis in breast cancer cells.

### Cytokine receptors are expressed in similar levels in breast cell lines

The high HER2-expressing cell lines SK-BR-3 and BT-474, the intermediate MCF-7 and T-47D and the low HER2-expressing MDA-MB-231 breast cancer cell lines, like the low HER2 normal immortalized MCF-10 breast cell line showed similar IFN-γ and TNF-α receptor expression by western blot analysis (Figure 12). This result demonstrates that the expression level of these two cytokine receptors is independent of the HER2 expression level. It is in accordance with reports that describe the action of these cytokines in the different phases of the normal breast. TNF-α has been involved proliferation, development and branching morphogenesis of the normal mammary gland (Lee et al., 2000 Endocrinology 141: 3764-3773). The receptor TNFR1 expression mediates TNF-α-induced proliferation of mammary epithelial cells, and TNFR2 activation induces casein accumulation (Varela et al., 1996 Endocrinology 137: 4915-4924). Similarly, the active form of IFN-γ interacts with its receptor expressed on the surface of almost all normal cells (Ealick et al., 1991 Science 252: 698-702; Farrar et al., 1993 Annu. Rev. Immunol. 11:571-611).

### Combined HER2 and HER3 blockage expression enhances Th1 cytokines TNF-α and IFN-γ senescence induction in breast cancer cells.

The previous results showing the enhancement of senescent and apoptosis phenotypes in high and intermediate HER2-expressing cell lines treated with the combination of cytokines led to studying the effect of knocking down HER2 and HER3 with siRNA. The therapeutic benefit of blocking HER2/HER3 signaling in breast cancer has been demonstrated by several studies (Lee-Hoeflich et al., 2008 Cancer Res. 14:5878-87; Berghoff et al., 2014 Breast 14: S0960-9776). Although the combined treatment of TNF-α and IFN-γ in HER3-depleted cells did not enhanced the number of senescent or apoptosis cells, the double knock down with HER2 and HER3 siRNA strongly increased the SA-β-gal staining (Figure 8A) and p15INK4b expression level (Figure 8B) in SK-BR-3 cells. At the same time, it was observed that a higher apoptosis induction of the cells treated with the double knock down and the cytokines by western blot of active caspase-3 (Figure 8A). Similar results were found in MCF-7 cells (Figure 13), BT-474 and T47D cells.

### Combined HER2 inhibition and HER2-HER3 dimerization inhibition enhances Th1 cytokines TNF-α and IFN-γ senescence induction and apoptosis in SK-BR-3 breast cancer cells

Trastuzumab and pertuzumab are antibodies that have been widely used in the clinic to treat HER2-positive breast cancer. To study the Th1 cytokines-induced senescence and apoptosis in a translational approach, experiments were designed to pretreat SK-BR-3 cells with trastuzumab and pertuzumab and then, the cells were treated with TNF-α and IFN-γ for 5 days at 37°C followed by 2 more passages without the cytokines and antibodies. It was observed that the amount of blue cells was highly increased in the cells that received the complete treatment compared to the cells treated with cytokines only, as measured by SA-β-gal staining (Figure 9A) and p15INK4b increased expression by western blot (Figure 9B). Interestingly, the double treatment also had a significant effect on the induction of apoptosis, both by increased active caspase 3 expression by western blot (Figure 9B) and by increased amount of anexin v and propidium iodide positive cells by flow cytometry analysis (Figure 9C).

### CD4⁺ Th1-mediated senescence and apoptosis of HER2-ovexpressing human breast cancer cells

Experiments were designed to co-culture HER2 Class II peptide-primed CD4⁺ T-cells with SK-BR-3 breast cancer cells using a transwell culture system to confirm that the cytokines produced by the immune system cells in vivo could also induce a specific senescence and apoptotic response in tumor cells. The cells were co-cultured for 5 days at 37°C and then cultured for 2 more passages in complete medium without immune system cells. The co-culture resulted in senescence and apoptosis of SK-BR-3 cells, evidenced by increased amount of SA-β-gal staining (Figure 10A) and increased expression of p15INK4b and cleaved caspase 3 (Figure 10B) detected by western blot. CD4⁺ T-cells primed either with immature dendritic cells (DCs) or mature DCs plus irrelevant Class II (BRAF or survivine) peptides were not able to induce senescence, nor apoptosis of SK-BR-3 cell (Figure 10).

The senescence and apoptosis observed was highly augmented in SK-BR-3 when we co-cultured them with HER2 Class II peptide-primed CD4⁺ T-cells in presence of trastuzumab and pertuzumab in the transwell. This result was clearly evidenced by increase SA-β-gal staining (Figure 10A) and p15INK4b and cleaved caspase 3 expression levels (Figure 10B).

As another approach, SK-BR-3 cells were co-cultured with the supernatant from CD4⁺ T cell-mature DC co-culture and a similar specific senescence response was observed. The Th1-elaborated cytokines IFN-γ and TNF-α obtained from CD4⁺ T cell-mature DC co-culture supernatants were previously confirmed using ELISA. By both experimental approaches, SK-BR-3 senescence and apoptosis could be partially rescued by neutralizing IFN-γ and TNF-α blocking antibodies (Figure 14).

It was also observed that the effect was dose-dependent as increasing number of immune system cells induced higher SA-β-gal staining, p15INK4b and cleaved caspase 3 expression levels in SK-BR-3 cells by both approaches.

### Th1 cytokines TNF-α and IFN-γ sensitize trastuzumab and pertuzumab resistant breast cancer cells to senescence and apoptosis induction

To continue unraveling the mechanism by which the Th1 cytokines could induce senescence and apoptosis, it is believed that TNF-α and IFN-γ could restore the sensitivity to trastuzumab and pertuzumab to breast cancer resistant cells. It was observed that the treatment with trastuzumab and pertuzumab could not prevent the activation of AKT in two resistant cell lines HCC-1419 (O'Brien et al., 2010 Mol Cancer Ther. 6:1489-502) and JIMT-1 (O'Brien et al., 2010 Mol Cancer Ther. 6:1489-502; Tanner et al., 2004 Mol Cancer Ther. 12:1585-92) contrary to T-47D sensitive cells (

Figure 16).

The treatment with TNF-α and IFN-γ induced senescence and apoptosis in a dose dependent manner in HCC-1419 and JIMT-1 cells. When the cells were treated with trastuzumab and pertuzumab, senescence and apoptosis could not be evidenced (Figure 11) even at higher doses (data not shown). However, the double treatment with cytokines and antibodies induced senescence by SA-β-gal assay (Figure 11A, 11C) and increase expression of p15INK4b (Figure 11B, 11D) in HCC-1419 and JIMT-1 cells. Moreover, the combination of cytokines and antibodies effectively induced cell death (Figure 11B, 11D) in HCC-1419 and JIMT-1 cells. This result demonstrates that the Th1 cytokines IFN-γ and TNF-α could revert the resistance to the therapeutic agents that is affecting cancer patients widely.

The disclosures of each and every patent, patent application, and publication cited herein are hereby incorporated herein by reference in their entirety. While this invention has been disclosed with reference to specific embodiments, it is apparent that other embodiments and variations of this invention may be devised by others skilled in the art without departing from the true spirit and scope of the invention. The appended claims are intended to be construed to include all such embodiments and equivalent variations.

## Claims

1. A dendritic cell vaccine in combination with an inhibitor of one or more of HER2 and HER3 for use in treating a cancer selected from the group consisting of: breast cancer, ovarian cancer, lung cancer, prostate cancer, colon cancer, melanoma, pancreatic cancer, gastrointestinal cancer, brain cancer, and any combination thereof.

2. The dendritic cell vaccine for use according to claim 1, wherein the inhibitor is an inhibitor of both HER-2 and HER-3 or a combination of a HER-2 inhibitor and a HER-3 inhibitor.

3. The dendritic cell vaccine for use according to claim 1 or 2, wherein the cancer is breast cancer.

4. The dendritic cell vaccine for use according to claim 3 wherein the breast cancer is negative for Estrogen Receptor (ER).

5. The dendritic cell vaccine for use according to claim 3 or 4, wherein the breast cancer is negative for ER and positive for HER-2.

6. The dendritic cell vaccine for use according to any one of claim 1-5, in association with TNF-α and IFN-γ, preferably TNF-α and IFN-γ are secreted from CD4 Th1 cells.

7. The dendritic cell vaccine for use according to any one of claims 1-6, in association with a chemokine modulator, preferably with a TLR agonist.

8. The dendritic cell vaccine for use according to claim 7, wherein the chemokine modulator is a TLR8 agonist.

9. The dendritic cell vaccine for use according to any one of claims 1-8, in association with a cancer medicament to treat the cancer or to reduce the risk of developing the cancer, wherein the cancer medicament is preferably selected from the group consisting of surgery, an anti-cancer agent, a chemotherapeutic agent, an immunotherapeutic agent, hormone therapy and combination thereof.

10. The dendritic cell vaccine for use according to any one of claims 1-9, wherein the inhibitor of one or more of HER2 and HER3 is selected from the group consisting of trastuzumab, pertuzumab and combination thereof.

11. The dendritic cell vaccine for use according to any one of claims 1-10, wherein the dendritic cell vaccine comprises an activated dendritic cell (DC) that has been contacted with at least one antigen and at least one TLR agonist.

12. The dendritic cell vaccine for use according to any one of claims 1-11, wherein the dendritic cell vaccine comprises an activated DC that has been contacted with a calcium ionophore and an activating agent.

13. The dendritic cell vaccine for use according to claim 12, wherein the calcium ionophore is selected from the group consisting of A23187 and ionomycin.

14. The dendritic cell vaccine for use according to any one of claims 1-13, wherein the dendritic cell vaccine is in the form of an injectable multi-dose antigen pulsed dendritic cell vaccine.

15. The dendritic cell vaccine for use according to claim 14, wherein multi-dose antigen pulsed dendritic cell vaccine is produced by collecting DCs in a single patient leukapheresis.


SEQUENCE LISTING

<110> CZERNIECKI, BRIAN J.
      KOSKI, GARY K.

<120> MANUFACTURING OF MULTI-DOSE INJECTION READY DENDRITIC CELL
      VACCINES AND COMBINATION THERAPY FOR BLOCKING HER2 AND HER3

<130> 319.004PCT

<140> PCT/US2015/041022
<141> 2015-07-17

<150> 62/029,774
<151> 2014-07-24

<150> 62/025,685
<151> 2014-07-17

<150> 62/165,445
<151> 2014-05-22

<150> 62/025,673
<151> 2014-07-17

<160> 12

<170> PatentIn version 3.5

<210> 1
<211> 19
<212> RNA
<213> Artificial Sequence

<220>
<223> Description of Artificial Sequence: Synthetic
      oligonucleotide

<400> 1
uggaagagau cacagguua                                                    19


<210> 2
<211> 19
<212> RNA
<213> Artificial Sequence

<220>
<223> Description of Artificial Sequence: Synthetic
      oligonucleotide

<400> 2
gagacccgcu gaacaauac                                                    19


<210> 3
<211> 19
<212> RNA
<213> Artificial Sequence

<220>
<223> Description of Artificial Sequence: Synthetic
      oligonucleotide

<400> 3
ggaggaaugc cgaguacug                                                    19

<210> 4
<211> 19
<212> RNA
<213> Artificial Sequence

<220>
<223> Description of Artificial Sequence: Synthetic
      oligonucleotide

<400> 4
gcucaucgcu cacaaccaa                                                    19

<210> 5
<211> 19
<212> RNA
<213> Artificial Sequence

<220>
<223> Description of Artificial Sequence: Synthetic
      oligonucleotide

<400> 5
gcgaugcuga gaaccaaua                                                    19

<210> 6
<211> 19
<212> RNA
<213> Artificial Sequence

<220>
<223> Description of Artificial Sequence: Synthetic
      oligonucleotide

<400> 6
agauugugcu cacgggaca                                                    19

<210> 7
<211> 19
<212> RNA
<213> Artificial Sequence

<220>
<223> Description of Artificial Sequence: Synthetic
      oligonucleotide

<400> 7
gcaguggauu cgagaagug                                                    19

<210> 8
<211> 18
<212> RNA
<213> Artificial Sequence

<220>

<223> Description of Artificial Sequence: Synthetic
      oligonucleotide

<400> 8
ucgucauguu gaacuaua                                                    18


<210> 9
<211> 19
<212> RNA
<213> Artificial Sequence

<220>
<223> Description of Artificial Sequence: Synthetic
      oligonucleotide

<400> 9
ugguuuacau guuuucuga                                                   19


<210> 10
<211> 19
<212> RNA
<213> Artificial Sequence

<220>
<223> Description of Artificial Sequence: Synthetic
      oligonucleotide

<400> 10
ugguuuacau guuuuccua                                                   19


<210> 11
<211> 19
<212> RNA
<213> Artificial Sequence

<220>
<223> Description of Artificial Sequence: Synthetic
      oligonucleotide

<400> 11
ugguuuacau gucgacuaa                                                   19


<210> 12
<211> 19
<212> RNA
<213> Artificial Sequence

<220>
<223> Description of Artificial Sequence: Synthetic
      oligonucleotide

<400> 12
ugguuuacau guuguguga                                                   19